# EUROPEAN PATENT APPLICATION

(11) **EP 4 714 430 A1**
(43) Date of publication of application: **25.03.2026**
(21) Application number: 24807016.1
(22) Date of filing: 26.04.2024
(51) Int. Cl.: A61K 8/894, A61K 8/891, A61Q 1/00, A61Q 1/04, A61Q 1/08, A61Q 5/06, A61Q 5/12, A61Q 17/04, A61Q 19/00

(54) **COSMETIC**

(30) Priority: 16.05.2023 JP 2023080546
(71) Applicant: SHIN-ETSU CHEMICAL CO., LTD., Tokyo 100-0005 (JP)
(72) Inventor: IMAI Taro, Annaka-shi, Gunma 379-0224 (JP); AKABANE Emi, Annaka-shi, Gunma 379-0224 (JP); MIYAUCHI Masaru, Tokyo 100-0005 (JP); KONISHI Masayuki, Tokyo 100-0005 (JP)
(74) Representative: Mewburn Ellis LLP
(86) International application number: PCT/JP2024/016412
(87) International publication number: WO 2024/237062

(57) **Abstract**

Provided is a cosmetic which contains a silicone gel obtained by swelling the following ingredient (a) with the following ingredient (b) and which has excellent emulsion stability, satisfactorily spreads when applied, and gives an excellent use feeling.
(a): A crosslinked glycerin-modified silicone obtained by crosslinking dimethylpolysiloxane with a glycerin chain.
(b): An oil having a 25°C dynamic viscosity of 0.5-5 mm²/s.

## Description

### TECHNICAL FIELD

The present invention relates to a cosmetic containing a silicone gel. In the present invention, a composition for cosmetics may be described as a cosmetic.

### BACKGROUND ART

A silicone gel activator has been used for the purpose of stabilizing a cosmetic, increasing the viscosity of an oil agent, or improving the usability. In Patent Document 1 and Patent Document 2, non-aqueous and emulsified cosmetics containing a cross-linked polyglycerol-modified silicone gel are known.

Patent Document 3 proposes a water-in-oil stick-shaped cosmetic using a cross-linked polyglycerol-modified silicone, and Patent Document 4 proposes a water break sunscreen cosmetic using a cross-linked polyglycerol-modified silicone. However, although stabilization by a component other than the cross-linked polyglycerol-modified silicone gel has been studied, an optimal base oil for the cross-linked polyglycerol-modified silicone gel has not been studied.

### PRIOR ART DOCUMENTS

### PATENT DOCUMENTS

| | |
|---|---|
| Patent Document 1: | WO 2004/024798 |
| Patent Document 2: | JP-A 2008-056687 |
| Patent Document 3: | WO 2017/163854 |
| Patent Document 4: | WO 2018/221174 |

### SUMMARY OF INVENTION

### TECHNICAL PROBLEM

The present invention has been made in view of the above circumstances and aims to provide a cosmetic with high emulsion stability, good spreadability during application, and high usability.

### SOLUTION TO PROBLEM

As a result of extensive studies to achieve the above object, the present inventors have completed the present invention by finding that a cosmetic containing a silicone gel in which a cross-linked glycerol-modified silicone produced by cross-linking dimethyl polysiloxane with a glycerol chain is swollen with a specific oil agent has high emulsion stability, good spreadability during application, and high usability, and can solve the above problems.

Accordingly, the present invention provides the following cosmetics.
1. A cosmetic containing a silicone gel in which the following component (a) is swollen with the following component (b):
   (a) a cross-linked glycerol-modified silicone in which dimethyl polysiloxane is cross-linked with a glycerol chain, and
   (b) an oil agent with a kinematic viscosity in the range of 0.5 to 5 mm²/s at 25°C.
2. The cosmetic according to 1, wherein the component (b) is a silicone oil with a kinematic viscosity in the range of 0.5 to 5 mm²/s at 25°C.
3. The cosmetic according to 2, wherein the component (b) is caprylyl methicone.
4. The cosmetic according to any one of 1 to 3, wherein the component (a) is a (dimethicone/polyglycerin-3) crosspolymer.
5. The cosmetic according to any one of 1 to 4, further containing (c) an ultraviolet absorber: 3.5% to 20% by mass.
6. The cosmetic according to any one of 1 to 5, further containing (d) a powder: 1% to 40% by mass.
7. The cosmetic according to any one of 1 to 6, wherein the cosmetic is a makeup cosmetic.

### ADVANTAGEOUS EFFECTS OF INVENTION

The present invention can provide a cosmetic with high emulsion stability, good spreadability during application, and high usability. In particular, even when an ultraviolet absorber, a powder, or the like is blended, a cosmetic with high emulsion stability, good spreadability during application, and high usability can be produced.

### DESCRIPTION OF EMBODIMENTS

The present invention will be described in detail below. In the present invention, the component name may be described as a cosmetic label name or International Nomenclature of Cosmetic Ingredient (INCI). When the cosmetic label name is equivalent to the INCI, the cosmetic label name or its English description may be omitted.

### [Silicone Gel]

A silicone gel of the present invention is a silicone gel in which the following component (a) is swollen with the following component (b), and the component (a) is a silicone gel produced by swelling the component (a) with the component (b) described later in advance and is blended in a cosmetic.

### [Component (a)]

In the component (a) of the present invention, a cross-linked glycerol-modified silicone in which dimethyl polysiloxane is cross-linked with a glycerol chain (hereinafter sometimes abbreviated to a cross-linked polyglycerol-modified silicone) is produced by cross-linking a linear dimethyl polysiloxane with a polyglycerol chain and can be used alone or in combination of two or more types thereof.

The cross-linked glycerol-modified silicone of the component (a) in which dimethyl polysiloxane is cross-linked with a glycerol chain is, for example, a cross-linked polyglycerol-modified silicone that can be produced by reacting a linear dimethyl hydrogen polysiloxane with a glycerol derivative having two or more alkenyl groups per molecule in the presence of a hydrosilylation reaction catalyst.

More specifically, the component (a) may be a (dimethicone/polyglycerin-3) crosspolymer (label name) (INCI: Dimethicone/Polyglycerin-3 Crosspolymer) or the like. The component (a) in a cosmetic of the present invention is a component for stabilizing the cosmetic.

The blending amount of the component (a) preferably ranges from 5% to 80% by mass, more preferably 15% to 60% by mass, still more preferably 20% to 50% by mass, of the entire silicone gel. It can be appropriately adjusted according to the compatibility with the component (b) and the ease of handling when blended in a cosmetic. When the blending amount of the component (a) is 5% or more by mass, a more sufficient effect can be produced when blended in a cosmetic. When the amount of the component (a) is 80% or less by mass, a gel is more easily formed.

The blending amount of the component (a) preferably ranges from 0.1% to 10% by mass, more preferably 0.1% to 4% by mass, still more preferably 0.2% to 3% by mass, particularly preferably 0.8% to 1.5% by mass, of the entire cosmetic. 0.1% or more by mass can result in a more stable emulsion, and 10% or less by mass can result in higher usability. In addition to the silicone gel, the component (a) may be separately contained in the cosmetic without losing the advantages of the present invention. In this case, it is preferable to swell by containing the liquid oil agent in an amount equal to or greater than its own weight. The above-described blending amount describes the blending amount of the entire component (a) in the entire cosmetic.

### [Component (b)]

The component (b) of the present invention is an oil agent with a kinematic viscosity in the range of 0.5 to 5 mm²/s at 25°C and can be used alone or in combination of two or more types thereof. The viscosity of the oil agent preferably ranges from 1.5 to 4.0 mm²/s, more preferably 2 to 3.5 mm²/s from the perspective of the affinity for the component (a) and usability. The oil agent is, for example, a silicone oil, a hydrocarbon oil, or the like. From the perspective of the affinity for the component (a), a silicone oil is preferred. In the present invention, the kinematic viscosity refers to a value measured at 25°C by a method using a Cannon-Fenske viscometer described in JIS Z 8803: 2011.

The silicone oil may be disiloxane (INCI) (0.7 mm²/s), cyclopentasiloxane (INCI) (4.0 mm²/s), dimethicone (INCI) with a kinematic viscosity in the range of 1.5 to 5 mm²/s at 25°C, trisiloxane (INCI) (1 mm²/s), an alkyl-modified silicone, such as methyl trimethicone (INCI) (1.5 mm²/s), ethyl trisiloxane (INCI) (1 mm²/s), ethyl methicone (INCI) (2.2 mm²/s), or caprylyl methicone (INCI) (3 mm²/s), or the like. In particular, caprylyl methicone is preferred because of its low viscosity, low irritation, high usability, and relatively good compatibility with another oil agent, such as a hydrocarbon oil. Examples of commercially available silicone oils include KF-96L-1cs, KF-96L-1.5cs, KF-96L-2cs, KF-4422, KF-4418, KF-995, and TMF-1.5 each manufactured by Shin-Etsu Chemical Co., Ltd.

The hydrocarbon oil may be isododecane (INCI) (1.9 mm²/s), undecane (INCI) (1.6 mm²/s), dodecane (INCI) (1.9 mm²/s), tridecane (INCI) (2.3 mm²/s), or the like.

The blending amount of the component (b) preferably ranges from 20% to 95% by mass, more preferably 40% to 85% by mass, still more preferably 50% to 80% by mass, of the entire silicone gel. It can be appropriately adjusted according to the compatibility with the component (b) and the ease of handling when blended in a cosmetic. When the blending amount of the component (b) is 20% or more by mass, a gel is more easily formed. When the blending amount of the component (b) is 95% or less by mass, a more sufficient effect can be produced when blended in a cosmetic.

The blending amount of the component (b) preferably ranges from 0.1% to 60% by mass, more preferably 1% to 50% by mass, still more preferably 5% to 40% by mass, particularly preferably 10% to 30% by mass, of the entire cosmetic. 0.1% or more by mass results in higher spreadability and applicability, and 60% or less by mass results in further improved usability. In addition to the silicone gel, the component (b) may be separately blended as an oily component without losing the advantages of the present invention. The above-described amount describes the blending amount of the entire component (b) in the entire cosmetic.

### [Other Components in Silicone Gel]

A silicone gel of the present invention is a cross-linked silicone gel containing the component (a) and the component (b), wherein the component (a) is swollen with the component (b). In the silicone gel, the total amount of the component (a) and the component (b) preferably ranges from 77% to 100% by mass, more preferably 80% to 100% by mass, still more preferably 90% to 100% by mass, particularly preferably 99% to 100% by mass, most preferably 100% by mass in which other components are not substantially blended.

A silicone gel of the present invention may contain a component other than the component (a) and the component (b) without losing the advantages of the present invention. It may be any component that can be blended in a cosmetic and may be a component that is blended in a cosmetic described later. Specific examples thereof include an antioxidant, a pH adjuster, a chelating agent, a surfactant, an oil agent other than the component (b), a preservative, and an oil-based thickener.

### [Physical Properties of Silicone Gel]

The silicone gel is a gel in which the component (a) is swollen with the component (b). In the present invention, the gel refers to a continuous paste state. The silicone gel can be produced by swelling the component (a) with the component (b), and the component (a) and the component (b) may be mixed with an ordinary stirrer and are preferably kneaded under shear force. This is because the component (a) has a three-dimensional cross-linked structure that does not dissolve in a solvent, and a gel-like composition with a smooth appearance can be produced by imparting sufficient dispersibility under shear force. The kneading treatment can be performed using, for example, a three-roll mill, a two-roll mill, a kneader, Masscolloider, a sand grinder, a colloid mill, a Gaulin homogenizer, a disperser, a high-shear mixer, or the like, and is preferably a method using a three-roll mill or a disperser.

### [Component (c)]

A cosmetic of the present invention may contain (c) an ultraviolet absorber. The ultraviolet absorber (c) may be any component that is usually used in cosmetics and can be used alone or in combination of two or more types thereof. The ultraviolet absorber may be homosalate (INCI), octocrylene (INCI), t-butyl methoxydibenzoylmethane (INCI: Butyl Methoxydibenzoylmethane), ethylhexyl salicylate (INCI: Ethylhexyl Salicylate), diethylamino hydroxybenzoyl hexyl benzoate (INCI: Diethylamino Hydroxybenzoyl Hexyl Benzoate), oxybenzone-6 (INCI: Benzophenone-6), oxybenzone-9 (INCI: Benzophenone-9), oxybenzone-1 (INCI: Benzophenone-1), polysilicone-15 (INCI), octyl dimethoxybenzylidene dioxoimidazolidine propionate (INCI: Ethylhexyl Dimethoxybenzylidene Dioxoimidazolidine Propionate), oxybenzone-2 (INCI: Benzophenone-2), terephthalylidene dicamphor sulfonic acid (INCI: Terephthalylidene Dicamphor Sulfonic Acid), ethylhexyl triazone (INCI), trimethoxycinnamic acid methyl bis(trimethylsiloxy)silyl isopentyl (INCI: Isopentyl Trimethoxycinnamate Trisiloxane), drometrizole trisiloxane (INCI), dimethyl PABA ethylhexyl (INCI: Ethylhexyl Dimethyl PABA), isopropyl p-methoxycinnamate (INCI: Isopropyl Methoxycinnamate), ethylhexyl methoxycinnamate (INCI: Ethylhexyl Methoxycinnamate), bisethylhexyloxyphenol methoxyphenyl triazine (INCI: Bis-Ethylhexyloxyphenol Methoxyphenyl Triazine), oxybenzone-3 (INCI: Benzophenone-3), oxybenzone-4 (INCI: Benzophenone-4), oxybenzone-5 (INCI: Benzophenone-5), phenylbenzimidazole sulfonic acid (INCI: Phenylbenzimidazole Sulfonic Acid), methylene bisbenzotriazolyl tetramethylbutylphenol (INCI: Methylene Bis-Benzotriazolyl Tetramethylbutylphenol), glyceryl ethylhexanoate dimethoxycinnamate (INCI: Glyceryl Ethylhexanoate Dimethoxycinnamate), glyceryl PABA (INCI: Glyceryl PABA), diisopropyl methyl cinnamate (INCI: Diisopropyl Methyl Cinnamate), cinoxate (INCI), ethylhexyl dimethoxybenzylidene dioxoimidazolidine propionate (INCI: Ethylhexyl Dimethoxybenzylidene Dioxoimidazolidine Propionate), or the like.

A UVA absorber (for example, diethylamino hydroxybenzoyl hexyl benzoate (INCI: Diethylamino Hydroxybenzoyl Hexyl Benzoate) or the like) and a UVB absorber (for example, ethylhexyl methoxycinnamate (INCI: Ethylhexyl Methoxycinnamate) or the like) can be used in combination, and each can be arbitrarily combined.

Among these, from the perspective of usability, one or two or more organic ultraviolet absorbers selected from ethylhexyl methoxycinnamate, diethylamino hydroxybenzoyl hexyl benzoate, ethylhexyl salicylate, polysilicone-15, t-butyl methoxydibenzoylmethane, oxybenzone, methylene bisbenzotriazolyl tetramethylbutylphenol, bisethylhexyloxyphenol methoxyphenyl triazine, homomenthyl salicylate, and octocrylene are preferred.

When the component (c) is blended, the amount thereof is preferably 3.5% to 20% by mass, more preferably 5% to 20% by mass, still more preferably 6% to 20% by mass, particularly preferably 7% to 12% by mass, most preferably 7.5% to 12% by mass, of the entire cosmetic.

### [(d) Powder]

A cosmetic of the present invention may contain (d) a powder. The powder may be a fine metal oxide powder, a hydrophobized color pigment, an inorganic powder, a metal powder, an organic powder, an inorganic/organic composite powder, or the like. These can be used alone or in combination of two or more types thereof. Those corresponding to the component (c) are excluded.

### • Fine Metal Oxide Powder

The fine metal oxide used in the present invention may be fine titanium dioxide (label name (INCI: Titanium Dioxide)), iron-containing titanium dioxide, zinc oxide (label name (INCI: Zinc Oxide)), cerium oxide (label name (INCI: Cerium Oxide)), or a complex thereof and can be used alone or in combination of two or more types thereof. The complex may be a composite powder of the metal oxide and another powder. From the perspective of the ultraviolet protection function and prevention of white residue, the average primary particle size is preferably 200 nm or less, more preferably 120 nm or less. The average primary particle size can be measured in a transmission electron micrograph or the like.

The fine metal oxide may be untreated or may be subjected to a known surface treatment generally used for cosmetics. Examples thereof include inorganic treatment, such as silica (label name (INCI: Silica)) coating, alumina (label name (INCI: Alumina)) coating, or Al hydroxide (label name (INCI: Aluminum Hydroxide)) coating, and organic treatment, such as a silane or silylation reagent, such as triethoxycaprylylsilane (label name (INCI: Triethoxycaprylylsilane)), a silicone oil, such as dimethicone (label name (INCI: Dimethicone)), hydrogen dimethicone (label name (INCI: Hydrogen Dimethicone)), or triethoxysilylethyl polydimethylsiloxyethyl hexyl dimethicone (label name (INCI: Triethoxysilylethyl Polydimethylsiloxyethyl Hexyl Dimethicone)), a wax, a paraffin, an organic fluorine compound, such as a perfluoroalkyl and a phosphate, a surfactant, an amino acid, such as N-acylglutamic acid, or a metallic soap, such as Al stearate (label name (INCI: Aluminum Stearate)) or Mg myristate (label name (INCI: Magnesium Myristate)). In particular, triethoxysilylethyl polydimethylsiloxyethyl hexyl dimethicone (label name (INCI: Triethoxysilylethyl Polydimethylsiloxyethyl Hexyl Dimethicone)) exhibits high dispersibility in both silicone and an oil agent and is therefore applied to a sunscreen or a foundation, and triethoxycaprylylsilane (label name (INCI: Triethoxycaprylylsilane)) and metallic soap treatment are preferred in terms of the compatibility with the component (b). These surface treatments may be used alone or in combination of two or more thereof depending on the purpose.

Commercial products of these surface-treated fine metal oxides can also be used. For example, fine titanium dioxide particles are commercially available under the trade names of STR-100C-LP, STR-100A-LP, STR-100W, STR-100W-LP, STR-100C-LF, and STR-40-LP (manufactured by Sakai Chemical Industry Co., Ltd.), MT-N1, MT-01, MT-05, MT-100Z, MT-100TV, MT-100AQ, MT-100WP, MTY-100SAS, MT-150EX, MT-500B, MT-505SAS, MT-700B, MT-700Z, MT-014Z, and SMT-500SAS (manufactured by Tayca Corporation), and ST-455, ST-455WS, ST-457ECS, and ST-495M (manufactured by Titan Kogyo, Ltd.). Fine zinc oxide particles are commercially available under the trade names of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, and FINEX-30S-LPT (manufactured by Sakai Chemical Industry Co., Ltd.), and MZ-150, MZ-200, MZ-300, MZ-306X, MZX-303S, MZX-303M, MZX-304OTS, MZ-500HP, MZ-505T, MZX-505HPS, MZY-505M, MZ-506X, MZY-203S, MZX-203OTS, MZY-210M3S, TMZ-HA1, and MZX-5080TS (manufactured by Tayca Corporation).

A dispersion in which these particles that absorb and scatter ultraviolet light are dispersed in an oil agent in advance can also be used. The oil agent may be a liquid silicone oil, hydrocarbon oil, ester oil, natural animal or vegetable oil, semi-synthetic oil, or fluorinated oil. Specific examples thereof include SPD series (trade name) manufactured by Shin-Etsu Chemical Co., Ltd., in particular, SPD-T5, T5L, Z5, Z5L, T6, Z6, T7, and Z7L.

### • Hydrophobized Color Pigment

The hydrophobized color pigment may be any pigment typically used for the purpose of coloring a cosmetic and may be any of color pigments, such as red iron oxide (label name (INCI: Iron Oxides)), yellow iron oxide (label name (INCI: Iron Oxides)), white titanium dioxide (label name (INCI: Titanium Dioxide)), black iron oxide (label name (INCI: Iron Oxides)), ultramarine (label name (INCI: Ultramarines)), Prussian blue (label name (INCI: Ferric Ferrocyanide, Ferric Ammonium Ferrocyanide)), manganese violet (label name (INCI: Manganese Violet)), cobalt titanate (label name (INCI: Cobalt Titanium Oxide)), chromium hydroxide (label name (INCI: Chromium Hydroxide Green)), chromium oxide (label name (INCI: Chromium Oxide Greens)), (Al/cobalt) oxide (label name (INCI: Cobalt Aluminum Oxide)), a (titanium/titanium dioxide) fired product (label name (INCI: Titanium/Titanium Dioxide)), (Li/cobalt) titanate (label name (INCI: Lithium Cobalt Titanate)), an (iron oxide/titanium dioxide) sinter (label name (IRON OXIDE/TITANIUM DIOXIDO SINTER)), a composite doped with a different type of metal, such as iron oxide doped titanium dioxide (label name (INCI: Iron Oxides, Titanium Dioxide)), titanium nitride (label name (INCI: Titanium Nitride)), iron(II) hydroxide (label name (INCI: Iron Hydroxide)), an inorganic brown pigment, such as γ-iron oxide, an inorganic yellowish pigment, such as ocher, a lake of a tar dye, and a lake of a natural dye.

The hydrophobized color pigment may have any shape, such as spherical, approximately spherical, rod-like, spindle-like, petal-like, strip-like, or an irregular shape, and may have any geometrical aspect that can impart a color to the cosmetic. From the perspective of hiding power, a pigment with a particle size, that is, a volume-average particle size (MV), in the range of 150 to 600 nm is preferred. The volume-average particle size can be measured by TEM. Less than 150 nm may result in a cosmetic with low coloring efficiency due to low hiding power, and more than 600 nm may result in poor usability.

Furthermore, a pigment according to the present invention may be partially or entirely surface-treated with an inorganic compound, such as alumina (label name (INCI: Alumina)), Al hydroxide (label name (INCI: Aluminum Hydroxide)), silica (label name (INCI: Silica)), or hydrous silica (label name (INCI: Hydrated Silica)).

The hydrophobization treatment of the hydrophobized color pigment means that the color pigment is surface-treated with a hydrophobizing agent. The surface hydrophobizing agent for a color pigment according to the present invention may be any agent that can impart hydrophobicity, and may be a silicone treatment agent, a wax, a paraffin, an organic fluorine compound, such as a perfluoroalkyl and a phosphate, a surfactant, an amino acid, such as N-acylglutamic acid, or a metallic soap, such as Al stearate (label name (INCI: Aluminum Stearate)) or Mg myristate (label name (INCI: Magnesium Myristate)).

Among these, a silicone treatment agent is preferably used, and examples thereof include a silane or a silylation reagent, such as triethoxycaprylylsilane (label name (INCI: Triethoxycaprylylsilane)) or trimethoxysilyl dimethicone (label name (INCI: Trimethoxysilyl dimethicone)), a silicone oil, such as dimethicone (label name (INCI: Dimethicone)), hydrogen dimethicone (label name (INCI: Hydrogen Dimethicone)), or triethoxysilylethyl polydimethylsiloxyethyl hexyl dimethicone (label name (INCI: Triethoxysilylethyl Polydimethylsiloxyethyl Hexyl Dimethicone)), and a silicone compound, such as an (acrylates/tridecyl acrylate/triethoxysilylpropyl methacrylate/dimethicone methacrylate) copolymer (label name (INCI: Acrylates/Tridecyl Acrylate/Triethoxysilylpropyl Methacrylate/Dimethicone Methacrylate Copolymer)) or an (acrylates/dimethicone) copolymer (label name (INCI: Acrylates/Dimethicone Copolymer)). As the silicone treatment agent, a silicone powder treatment agent described in Japanese Patent No. 3912961 is suitably used, and particularly, triethoxysilylethyl polydimethylsiloxyethyl hexyl dimethicone (label name (INCI: Triethoxysilylethyl Polydimethylsiloxyethyl Hexyl Dimethicone)), which is dimethyl polysiloxane having a triethoxysilyl group, a polydimethylsiloxyethyl group, and a hexyl group in a side chain, and the like are effectively used from the perspective of exhibiting high affinity even when a dispersion medium for dispersing the highly hydrophobized color pigment has a mixed composition of a silicone, a hydrocarbon, and the like. These surface hydrophobizing agents can be used alone or in combination of two or more types thereof.

Examples of commercially available color pigments subjected to hydrophobic surface treatment include KTP-09 series, particularly, KTP-09W, KTP-09R, KTP-09Y, and KTP-09B (manufactured by Shin-Etsu Chemical Co., Ltd.).

### • Inorganic Powder

The inorganic powder may be fine particles composed of zirconium oxide (label name (INCI: Zirconium Dioxide)), zinc oxide (label name (INCI: Zinc Oxide)), cerium oxide (label name (INCI: Cerium Oxide)), Mg oxide (label name (INCI: Magnesium Oxide)), Ba sulfate (label name (INCI: Barium Sulfate)), calcium sulfate (label name (INCI: Calcium Sulfate)), Mg sulfate (label name (INCI: Magnesium Sulfate)), Ca carbonate (label name (INCI: Calcium Carbonate)), Mg carbonate (label name (INCI: Magnesium Carbonate)), talc (label name (INCI: Talc)), platy talc (label name (INCI: Talc)), mica (label name (INCI: Mica)), kaolin (label name (INCI: Kaolin)), sericite (label name (INCI: Mica)), synthetic fluorophlogopite (label name (INCI: Synthetic Fluorphlogopite), biotite (label name (INCI: Biotite), K silicate (label name (INCI: Potassium Silicate)), silica (label name (INCI: Silica), fumed silica, Al silicate (label name (INCI: Aluminum Silicate)), Mg silicate (label name (INCI: Magnesium Silicate)), silicic acid (Al/Mg) (label name (INCI: Magnesium Aluminum Silicate)), Ca silicate (label name (INCI: Calcium Silicate)), (Al/Ca/Na) silicate (label name (INCI: Aluminum Calcium Sodium Silicate)), (Li/Mg/Na) silicate (label name (INCI: Lithium Magnesium Sodium Silicate)) (Na/Mg) silicate (label name (INCI: Sodium Magnesium Silicate)), (Ca/Al) borosilicate (label name (INCI: Calcium Aluminum Borosilicate)), (Ca/Na) borosilicate (label name (INCI: Calcium Sodium Borosilicate)), hydroxyapatite (label name (INCI: Hydroxyapatite)), bentonite (label name (INCI: Bentonite)), montmorillonite (label name (INCI: Montmorillonite)), hectorite (label name (INCI: Hectorite)), zeolite (label name (INCI: Zeolite)), alumina (label name (INCI: Alumina)), Al hydroxide (label name (INCI: Aluminum Hydroxide)), boron nitride (label name (INCI: Boron Nitride)), glass (label name (INCI: Glass)), or the like. Furthermore, examples of an inorganic color pearl pigment include a pearl, such as mica coated with titanium dioxide, and a pearl pigment, such as bismuth oxychloride (label name (INCI: Bismuth Oxychloride)), bismuth oxychloride (label name (INCI: Bismuth Oxychloride)) coated with titanium dioxide (label name (INCI: Titanium Dioxide)), talc (label name (INCI: Talc)) coated with titanium oxide (label name (INCI: Titanium Dioxide)), argentine, and coloring mica coated with titanium dioxide (label name (INCI: Titanium Dioxide)), and the inorganic color pearl pigment may be untreated or may be subjected to a known surface treatment generally used for cosmetics.

### • Metal Powder

The metal powder is, for example, fine metal particles composed of aluminum (label name (INCI: Aluminum Powder)), copper (label name (INCI: Copper Powder)), silver (label name (INCI: Silver Powder)), or the like.

### • Organic Powder

The organic powder is, for example, a powder composed of silicone, polyamide, a poly(acrylic acid)-acrylic acid ester, polyester, polyethylene, polypropylene, polystyrene, a styrene-acrylic acid copolymer, a divinylbenzene-styrene copolymer, polyurethane, a vinyl resin, a urea resin, a melamine resin, benzoguanamine, polymethylbenzoguanamine, tetrafluoroethylene, poly(methyl methacrylate) (for example, poly(methyl methacrylate) or the like), cellulose, silk, nylon, a phenolic resin, an epoxy resin, polycarbonate, or the like. In particular, the silicone may be silicone resin particles (specific examples thereof include polymethylsilsesquioxane (label name (INCI: Polymethylsilsesquioxane)), commercial products thereof including KMP-590 and KMP-591 each manufactured by Shin-Etsu Chemical Co., Ltd.; methylphenyl polysilsesquioxane (label name (INCI: Methyl/Phenyl Polysilsesquioxane), commercial products thereof including KMP-592 manufactured by Shin-Etsu Chemical Co., Ltd.)); a silicone rubber powder (specific examples thereof include KMP-597 and KMP-598 each manufactured by Shin-Etsu Chemical Co., Ltd.); a silicone resin-coated silicone rubber powder (specific examples thereof include a (vinyl dimethicone/methicone silsesquioxane) crosspolymer (label name (INCI: Vinyl Dimethicone/Methicone Silsesquioxane Crosspolymer)), commercial products thereof including KSP-100, KSP-101, KSP-102, and KSP-105 each manufactured by Shin-Etsu Chemical Co., Ltd.); a (diphenyl dimethicone/vinyl diphenyl dimethicone/silsesquioxane) crosspolymer (label name (INCI: Diphenyl Dimethicone/Vinyl Diphenyl Dimethicone/Silsesquioxane Crosspolymer), commercial products thereof including KSP-300 manufactured by Shin-Etsu Chemical Co., Ltd.); a polysilicone-1 crosspolymer (label name (INCI: Polysilicone-1 Crosspolymer), commercial products thereof including KSP-411 manufactured by Shin-Etsu Chemical Co., Ltd.); polysilicone-22 (label name (INCI: Polysilicone-22), commercial products thereof including KSP-441 manufactured by Shin-Etsu Chemical Co., Ltd.); or the like. Specific examples of a hydrophilized silicone resin-coated silicone rubber powder include KSP-100W manufactured by Shin-Etsu Chemical Co., Ltd. It may be dispersed in water or oil in advance (specific examples thereof include KM-9729, KM-440, and KSG-016F each manufactured by Shin-Etsu Chemical Co., Ltd.). Furthermore, a metallic soap and the like are also mentioned, and specific examples thereof include a powder composed of zinc stearate (label name (INCI: Zinc Stearate)), Al stearate (label name (INCI: Aluminum Stearate)), Ca stearate (label name (INCI: Calcium Stearate)), Mg stearate (label name (INCI: Magnesium Stearate)), zinc myristate (label name (INCI: Zinc Myristate)), Mg myristate (label name (INCI: Magnesium Myristate)), (zinc/Na) cetyl phosphate (label name (INCI: Sodium Zinc Cetyl Phosphate)), K cetyl phosphate (label name (INCI: Potassium Cetyl Phosphate)), or the like. An organic dye may also be mentioned, and specific examples thereof include tar dyes, such as red 3, red 104(1) (label name (INCI: Red 28, Red 28 Lake)), red 106, red 201 (label name (INCI: Red 6)), red 202 (label name (INCI: Red 7)), red 204, red 205, red 220 (label name (INCI: Red 34)), red 226 (label name (INCI: Red 30)), red 227 (label name (INCI: Red 33, RED 33 Lake)), red 228 (label name (INCI: Red 36)), red 230(1) (label name (INCI: Red 22, Red 22 Lake)), red 230(2), red 401, red 505, yellow 4 (label name (INCI: Yellow 5)), yellow 5 (label name (INCI: Yellow 6, Yellow 6 Lake)), yellow 202(1) (label name (INCI: Yellow 8)), yellow 203 (label name (INCI: Yellow 10, Yellow 10 Lake)), yellow 204 (label name (INCI: Yellow 11)), yellow 401 (label name (INCI:)), blue 1 (label name (INCI: Blue 1, Blue 1 Lake)), blue 2, blue 201, blue 205 (label name (INCI: Blue 4)) blue 404, green 3 (label name (INCI: Green 3, Green 3 Lake)), green 201 (label name (INCI: Green 5)), green 202 (label name (INCI: Green 6)), green 204 (label name (INCI: Green 8)), green 205, orange 201 (label name (INCI: Orange 5)), orange 203 (label name (INCI: Pigment Orange 5)), orange 204, orange 205 (label name (INCI: Orange 4, Orange 4 Lake)), orange 206 (label name (INCI: Orange 10)), and orange 207 (label name (INCI: Orange 11)), and natural dyes, such as cochineal (label name (INCI: Cochineal)), laccaic acid (label name (INCI: Laccaic Acid)), safflower red (label name (INCI: Carthamus Tinctorius (Safflower) Flower Extract)), a Lithospermum erythrorhizon root extract (label name (INCI: Lithospermum Officinale Root Extract)), gardenia yellow, and gardenia blue (label name (INCI: Hydrolyzed Gardenia Florida Extract)).

### • Inorganic/Organic Composite Powder

The inorganic/organic composite powder is, for example, a composite powder in which the surface of an inorganic powder is coated with an organic powder by a publicly known method.

Among these, a fine metal oxide powder and a hydrophobized color pigment are particularly preferred. When the fine metal oxide powder is blended, the blending amount thereof preferably ranges from 1% to 25% by mass, more preferably 3% to 15% by mass, still more preferably 6% to 10% by mass, of the entire cosmetic. A blending amount of 1% or more by mass results in a more sufficient ultraviolet-shielding effect. When the hydrophobized color pigment is blended, the blending amount thereof preferably ranges from 1% to 25% by mass, more preferably 3% to 15% by mass, still more preferably 6% to 10% by mass, of the entire cosmetic. A blending amount of 1% or more by mass enhances the masking and coloring effects.

When the component (d) is blended, the blending amount thereof preferably ranges from 1% to 40% by mass, more preferably 1% to 25% by mass, still more preferably 3.0% to 20% by mass, particularly preferably 6.0% to 20% by mass, most particularly preferably 12% to 20% by mass, of the entire cosmetic. A blending amount of 40% or less by mass can result in a further stabilized cosmetic.

The combined use of the components (c) and (d) can achieve both the ultraviolet protection effect and the masking effect. Furthermore, a large total blending amount of the components (c) and (d) results in a higher ultraviolet protection effect but makes formulation design difficult. The present invention can provide a cosmetic with a good feel even if the components (c) and (d) are blended in higher amounts. The total blending amount of the components (c) and (d) preferably ranges from 4.5% to 45% by mass, more preferably 6.0% to 30% by mass, still more preferably 12% to 30% by mass, particularly preferably 19% to 30% by mass, of the entire cosmetic.

### [Cosmetic]

A cosmetic of the present invention contains the silicone gel, and the present invention is applied to various cosmetics. Examples thereof include a cosmetic for external use on the skin, such as a skincare cosmetic, a makeup cosmetic, an antiperspirant cosmetic, or an ultraviolet protection cosmetic, a cosmetic for external use on the hair, such as a hair cosmetic, and a cosmetic for nails. The skincare cosmetic is, for example, a lotion, a milky lotion, a cream, a cleansing, a pack, an oil liquid, a massage cosmetic, a beauty liquid, a beauty oil, a detergent, a deodorant, a hand cream, a lip cream, a wrinkle concealer, or the like. The makeup cosmetic is, for example, a makeup base, a foundation, a concealer, a white powder, a cheek rouge, an eye color, an eye shadow, a mascara, an eyeliner, an eyebrow, a lipstick, or the like. The antiperspirant cosmetic is, for example, an antiperspirant cosmetic of roll-on type, cream type, solution type, stick type, or the like. The ultraviolet protection cosmetic is, for example, a sunscreen oil, a sunscreen emulsion, a sunscreen cream, a preparation produced by imparting a sunscreen effect to the makeup cosmetic, or the like. The hair cosmetic is, for example, a shampoo, a rinse, a hair treatment, a setting agent, a hair dye, a hair fragrance, or the like.

Among these, a makeup cosmetic in which a color pigment is blended in a large amount, such as a foundation or a concealer, or a makeup cosmetic to which a sunscreen effect is imparted is particularly preferred.

The form of a cosmetic of the present invention may be, for example, any of a powder, an oil-based liquid, an emulsion type, such as a water-in-oil emulsion, an oil-in-water emulsion, a non-aqueous emulsion, or a multi-emulsion, such as a W/O/W type or an O/W/O type, and the like. Furthermore, the properties of a cosmetic of the present invention can be selected from various properties, such as liquid, milky liquid, cream, solid, paste, gel, powder, pressed, multilayer, mousse, spray, stick, and pencil. Among these, an emulsion type is preferred, and a water-in-oil emulsion is most preferred. The water-in-oil emulsion may be a viscous material or a solid material. The viscous material preferably has a viscosity in the range of 5,000 to 200,000 mPa·s. The viscosity is a value at 25°C measured by a method described in JIS K 7117-1: 1999 using a B-type rotational viscometer (for example, TVB-10 type, manufactured by Toki Sangyo Co., Ltd.).

A cosmetic of the present invention may contain various components used in ordinary cosmetics without losing the advantages of the present invention. For example, it may contain (1) an oil agent other than (b), (2) a surfactant other than (a), (3) a composition composed of a cross-linked organopolysiloxane and an oil agent that is liquid at room temperature, (4) a film-forming agent, (5) an aqueous component, and (6) other additive agents. These can be used alone or in combination of two or more types thereof in an appropriate amount.

### (1) Oil Agent other than (b)

The oil agent may be any of a solid, a semi-solid, and a liquid at room temperature (25°C) and is, for example, a silicone oil, a silicone wax, a natural animal or plant oil or fat, a semi-synthetic fat or oil, a hydrocarbon oil, a higher alcohol, a fatty acid, an ester oil, a fluorinated oil agent, or the like.

### • Silicone Oil

The silicone oil may be any silicone oil other than the silicone oil with a kinematic viscosity in the range of 0.5 to 5 mm²/s at 25°C as the component (b). Examples thereof include an alkyl-modified silicone, such as dimethicone (INCI) or hexyl dimethicone (INCI), a phenyl-modified silicone, such as phenyl trimethicone (INCI), diphenyl dimethicone (INCI), diphenylsiloxyphenyl trimethicone (INCI), or tetraphenyldimethyl disiloxane (INCI), an amino-modified organopolysiloxane, such as amodimethicone (INCI) or aminopropyl dimethicone (INCI), a pyrrolidone-modified organopolysiloxane, such as PCA dimethicone (INCI), a silicone rubber, such as pyrrolidonecarboxylic acid-modified organopolysiloxane, a gum-like dimethyl polysiloxane with a high degree of polymerization, gum-like amino-modified organopolysiloxane, or a gum-like dimethylsiloxane-methylphenylsiloxane copolymer, a low-viscosity organopolysiloxane solution of a silicone gum or rubber, an amino acid-modified silicone, a fluorine-modified silicone, a silicone resin, and a dissolved product of a silicone resin. Among these silicone oils, the blending amount of a linear dimethyl polysiloxane with a kinematic viscosity of more than 5 mm²/s and 100 mm²/s or less at 25°C is preferably 2.8% or less by mass, more preferably 0.5% or less by mass, of the entire cosmetic, and it is still more preferable that the linear dimethyl polysiloxane is not substantially blended. When the blending amount is 2.8% or less by mass, the cosmetic can be stably emulsified, has a light touch, high usability, and good applicability (spreadability). Examples of commercially available silicone oils include KF-54, KF-54HV, and KF-56A each manufactured by Shin-Etsu Chemical Co., Ltd.

### • Solid Oily Component

In the present invention, when it is desired to solidify the cosmetic, an oily component that is solid at 25°C is preferably blended. The oily component that is solid at 25°C preferably has a melting point of 40°C or more, more preferably 60°C to 110°C, may be a wax, a hydrocarbon, an ester, a higher alcohol, or a higher fatty acid, and may be any raw material that can be blended in ordinary cosmetics. Specific examples thereof include vegetable wax, such as carnauba wax (INCI: Copernicia Cerifera (Carnauba) Wax), sugarcane wax, candelilla wax (INCI: Euphorbia Cerifera (Candelilla) Wax), refined candelilla wax, rice wax, Japan wax, jojoba wax, kapok wax, rice bran wax, bayberry wax, shea butter, cocoa butter, Japan wax (INCI: Rhus Succedanea Fruit Wax), montan wax (INCI: Montan Wax), or hydrogenated castor oil isostearate, an animal wax, such as beeswax, beef tallow, beef bone fat, lard (INCI: Lard), horse fat (INCI: Horse Fat), mutton tallow, lanolin (INCI: Lanolin), Chinese wax, shellac wax, or spermaceti, a semi-synthetic wax, such as a lanolin ester, a lanolin fatty acid ester, or a beeswax ester, a hydrogenated oil, such as hydrogenated castor oil or hydrogenated coconut oil, a hydrocarbon wax, such as solid paraffin, polyethylene, ceresin, ozokerite, or microcrystalline wax, a wax ester, such as synthetic beeswax, an amino acid stearyl alcohol, such as dioctyldodecyl lauroylglutamate, dioctyldodecyl lauroylglutamate, or dioctyldodecyl lauroylglutamate, a fatty acid, such as stearic acid or behenic acid, a silicone wax, such as an acrylic silicone resin of an acrylic-silicone graft or block copolymer (an acrylic-silicone graft copolymer KP-561P, 562P, or the like each manufactured by Shin-Etsu Chemical Co., Ltd.), and a derivative thereof, and one or two or more selected from these are preferred.

### • Natural Animal or Vegetable Oil Agent and Semi-Synthetic Oil Agent

Examples of a natural animal or vegetable oil agent and a semi-synthetic oil agent include a germ oil, such as avocado oil (label name (INCI: Persea Gratissima (Avocado) Oil)), linseed oil (label name (INCI: Linum Usitatissimum (Linseed) Seed Oil)), almond oil (label name (INCI: Prunus Amygdalus Dulcis (Sweet Almond) Oil)), perilla oil (label name), olive oil (label name (INCI: Olea Europaea (Olive) Fruit Oil)), Torreya californica oil (label name (INCI: Torreya Californica (California Nutmeg) Oil)), citronella oil (label name (INCI: Cymbopogon Nardus (Citronella) Oil)), Khaya seed oil (label name (INCI: Torreya Nucifera Seed Oil)), apricot kernel oil (label name (INCI: Kyounin Yu)), wheat germ oil (label name (INCI: Triticum Vulgare (Wheat) Germ Oil)), sesame oil (label name (INCI: Sesamum Indicum (Sesame) Seed Oil)), rice germ oil (label name (INCI: Oryza Sativa (Rice) Germ Oil)), rice bran oil (label name (INCI: Oryza Sativa (Rice) Bran Oil)), sasanqua oil (label name (INCI: Camellia Kissi Seed Oil)), safflower oil (label name (INCI: Carthamus Tinctorius (Safflower) Seed Oil)), soybean oil (label name (INCI: Glycine Soja(Soybean)Oil)), Camellia sinensis seed oil (label name (INCI: Camellia Sinensis Seed Oil)), camellia oil (label name (INCI: Camellia Japonica Seed Oil)), evening primrose oil (label name (INCI: Oenothera Biennis (Evening Primrose) Oil)), rapeseed oil (label name), or corn germ oil (label name (INCI: Zea Mays (Corn) Germ Oil)), a natural vegetable oil, such as persic oil (label name), palm oil (label name (INCI: Elaeis Guineensis (Palm) Oil)), palm kernel oil (label name (INCI: Elaeis Guineensis (Palm) Kernel Oil)), castor oil (label name (INCI: Ricinus Communis (Castor) Seed Oil)), sunflower seed oil (label name (INCI: Helianthus Annuus (Sunflower) Seed Oil)), grape seed oil (label name (INCI: Vitis Vinifera (Grape) Seed Oil)), jojoba seed oil (label name (INCI: Simmondsia Chinensis (Jojoba) Seed Oil)), macadamia seed oil (label name (INCI: Macadamia Ternifolia Seed Oil)), meadowfoam oil (label name (INCI: Limnanthes Alba (Meadowfoam) Seed Oil)), cotton seed oil (label name (INCI: Gossypium Herbaceum (Cotton) Seed Oil)), coconut oil (label name (INCI: Cocos Nucifera (Coconut) Oil)), or peanut oil (label name (INCI: Arachis Hypogaea (Peanut) Oil)), a natural animal oil, such as shark liver oil (label name (INCI: Shark Liver Oil)), cod liver oil (label name (INCI: Cod Liver Oil)), fish liver oil (label name (INCI: Fish Liver Oil)), turtle oil (label name (INCI: Turtle Oil)), mink oil (label name (INCI: Mink Oil)), or yolk oil (label name (INCI: Egg Oil)), and a semi-synthetic fat or oil, such as hydrogenated coconut oil (label name (INCI: Hydrogenated Coconut Oil)) or liquid lanolin (label name (INCI: Lanolin Oil)).

### • Hydrocarbon Oil

The hydrocarbon oil is, for example, a linear or branched hydrocarbon oil. Specific examples thereof include an isoparaffin, such as an olefin oligomer (INCI) or a (C13, 14) isoparaffin (INCI), and an alkane, such as hydrogenated polyisobutene (label name (INCI: Hydrogenated Polyisobutene)), squalane (INCI), mineral oil (INCI), a coconut alkane (INCI), or a (C13-15) alkane (INCI).

### • Higher Alcohol

The higher alcohol is, for example, a linear saturated alcohol with 6 or more carbon atoms, such as lauryl alcohol (INCI), hexyldecanol (INCI), oleyl alcohol (INCI), isostearyl alcohol (INCI), octyldodecanol (INCI), decyltetradecanol (INCI), myristyl alcohol (INCI), cetyl alcohol (INCI), stearyl alcohol (INCI), or behenyl alcohol (INCI), or batyl alcohol (INCI). Also included are sterols, such as cholesterol (INCI), sitosterol (label name (INCI: Beta-Sitosterol)), phytosterol (INCI), lanosterol (INCI), and the like.

### • Ester Oil

The ester oil may be diisobutyl adipate (label name (INCI: Diisobutyl Adipate)), dihexyldecyl adipate (label name), diheptylundecyl adipate (label name (INCI: Diheptylundecyl Adipate)), a alkyl glycol monoisostearate, such as isostearyl isostearate (label name (INCI: Isostearyl Isostearate)), isocetyl isostearate (label name (INCI: Isocetyl Isostearate)), trimethylolpropane triisostearate (label name (INCI: Trimethylolpropane Triisostearate)), glycol diethylhexanoate (label name (INCI: Glycol Diethylhexanoate)), cetyl ethylhexanoate (label name (INCI: Cetyl Ethylhexanoate)), trimethylolpropane triethylhexanoate (label name (INCI: Trimethylolpropane Triethylhexanoate)), pentaerythrityl tetraethylhexanoate (label name (INCI: Pentaerythrityl Tetraethylhexanoate)), an octyldodecyl ester, such as octyldodecyl stearoyl stearate (label name (INCI: Octyldodecyl Stearoyl Stearate)), oleyl oleate (label name (INCI: Oleyl Oleate)), octyldodecyl oleate (label name (INCI: Octyldodecyl Oleate)), decyl oleate (label name (INCI: Decyl Oleate)), neopentyl glycol diethylhexanoate (label name (INCI: Neopentyl Glycol Diethylhexanoate)), Neopentyl glycol dicaprate (label name (INCI: Neopentyl Glycol Dicaprate)), diisostearyl malate (label name (INCI: Diisostearyl Malate)), triethyl citrate (label name (INCI: Triethyl Citrate)), diethylhexyl succinate (label name (INCI: Diethylhexyl Succinate)), amyl acetate (label name (INCI: Amyl Acetate)), ethyl acetate (label name (INCI: Ethyl Acetate)), butyl acetate (label name (INCI: Butyl Acetate)), isocetyl stearate (label name (INCI: Isocetyl Stearate)), butyl stearate (label name (INCI: Butyl Stearate)), diisopropyl sebacate (label name (INCI: Diisopropyl Sebacate)), diethylhexyl sebacate (label name (INCI: Diethylhexyl Sebacate)), cetyl lactate (label name (INCI: Cetyl Lactate)), myristyl lactate (label name (INCI: Myristyl Lactate)), isononyl isononanoate (label name (INCI: Isononyl Isononanoate)), isotridecyl isononanoate (label name (INCI: Isotridecyl Isononanoate)), a palmitate, such as isopropyl palmitate (label name (INCI: Isopropyl Palmitate)), ethylhexyl palmitate (label name (INCI: Ethylhexyl Isopalmitate)), or hexyldecyl palmitate (label name (INCI: Isocetyl Palmitate, Hexyldecyl Palmitate)), cholesteryl (label name (INCI: Cholesteryl Hydroxystearate)), a myristate, such as isopropyl myristate (label name (INCI: Isopropyl Myristate)), octyldodecyl myristate (label name (INCI: Octyldodecyl Myristate)), or myristyl myristate (label name (INCI: Myristyl Myristate)), ethylhexyl laurate (label name (INCI: Ethylhexyl Laurate)), hexyl laurate (label name (INCI: Hexyl Laurate)), dioctyldodecyl lauroyl glutamate (label name (INCI: Dioctyldodecyl Lauroyl Glutamate)), isopropyl lauroyl sarcosinate (label name (INCI: Isopropyl Lauroyl Sarcosinate)), a cocoalkyl (caprylate/caprate) (label name (INCI: Coco-Caprylate/Caprate)), or the like.

Among the ester oils, a glyceride oil may be triethylhexanoin (INCI), (caprylic/capric) triglyceride (label name (INCI: Caprylic/Capric Triglyceride)), cocoglyceryl (INCI), (caprylic/capric/succinic) triglyceride (label name (INCI: Caprylic/Capric/Succinic Triglyceride)), (caprylic/capric) glyceride (label name (INCI: Caprylic/Capric Glycerides)), or the like.

### (2) Surfactant other than (a)

The surfactant is nonionic, anionic, cationic, or amphoteric and may be any surfactant that can be used in ordinary cosmetics. Among these surfactants, one or two or more selected from non-cross-linked silicone surfactants are preferred because a cosmetic with high emulsion stability can be produced. In any case, when a surfactant other than (a) is blended, the blending amount thereof preferably ranges from 0.1% to 20% by mass of the entire cosmetic. It is preferred because 0.1% or more by mass can result in a sufficient dispersion or emulsification function, and 20% or less by mass results in a cosmetic without a sticky feel. The HLB of the surfactant is preferably, but not limited to, in the range of 2 to 14.5 for the purpose of maintaining the water resistance of the cosmetic.

The non-cross-linked silicone surfactant is a silicone surfactant in which part of the methyl groups in a linear or branched silicone main chain is substituted with a hydrophilic group of poly(ethylene glycol), polyglycerol, or the like, and specific examples thereof preferably include a linear or branched polyoxyethylene-modified organopolysiloxane, a linear or branched polyoxyethylene-polyoxypropylene-modified organopolysiloxane, a linear or branched polyoxyethylene-alkyl-co-modified organopolysiloxane, a linear or branched polyoxyethylene-polyoxypropylene-alkyl-co-modified organopolysiloxane, a linear or branched polyglycerol-modified organopolysiloxane, a linear or branched polyglycerol-alkyl-co-modified organopolysiloxane, and a linear or branched pyrrolidone-modified organopolysiloxane. PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 disiloxane dimethicone (INCI), polyglyceryl-3 polydimethylsiloxyethyl dimethicone (INCI), lauryl polyglyceryl-3 polydimethylsiloxyethyl dimethicone (INCI), bisbutyl dimethicone polyglyceryl-3 (INCI), and the like are mentioned. Examples of commercial products thereof include KF-6011, KF-6011P, KF-6012, KF-6015, KF-6017, KF-6043, KF-6028, KF-6038, KF-6048, KF-6100, KF-6104, KF-6106, KF-6105, and KF-6115 each manufactured by Shin-Etsu Chemical Co., Ltd.

From the perspective of compatibility with the component (a), a linear or branched polyglycerol-modified organopolysiloxane and a linear or branched polyglycerol-alkyl-co-modified organopolysiloxane are preferred.

A cross-linked silicone surfactant other than the component (a) may be blended. Examples thereof include cross-linked polyether-modified silicones, such as a (dimethicone/(PEG-10/15)) crosspolymer (INCI), a (PEG-15/lauryl dimethicone) crosspolymer (INCI), a (PEG-10/lauryl dimethicone) crosspolymer (INCI), and a (PEG-15/lauryl polydimethylsiloxyethyl dimethicone) crosspolymer (INCI). When a cross-linked silicone surfactant is used, in a composition composed of the cross-linked silicone surfactant and an oil agent that is liquid at room temperature, the cross-linked silicone surfactant preferably contains the liquid oil agent in an amount equal to or greater than its own weight and swells in the liquid oil.

The liquid oil agent may be the component (b) or (1) a liquid silicone oil, a hydrocarbon oil, an ester oil, a natural animal or vegetable oil, a semi-synthetic oil, or a fluorinated oil in the oil agent as an optional component, for example, cyclopentasiloxane (INCI), dimethicone (INCI), mineral oil (INCI), isododecane (INCI), isohexadecane (INCI), triethylhexanoin (INCI), isotridecyl isononanoate (label name (INCI: Isotridecyl Isononanoate)), squalane (INCI), or the like. Examples of commercially available cross-linked silicone surfactants that contain a liquid oil agent and swell include KSG-210, KSG-240, KSG-270, KSG-310, KSG-320, KSG-330, KSG-340, KSG-320Z, and KSG-350Z each manufactured by Shin-Etsu Chemical Co., Ltd.

### (3) Composition Composed of Cross-Linked Organopolysiloxane and Oil Agent That Is Liquid at Room Temperature

In a composition composed of a cross-linked organopolysiloxane and an oil agent that is liquid at room temperature, the cross-linked organopolysiloxane preferably contains the liquid oil agent in an amount equal to or greater than its own weight and swells in the liquid oil. The liquid oil agent may be the component (b) or (1) a liquid silicone oil, a hydrocarbon oil, an ester oil, a natural animal or vegetable oil, a semi-synthetic oil, or a fluorinated oil in the oil agent as an optional component, for example, cyclopentasiloxane (INCI), dimethicone (INCI), mineral oil (INCI), isododecane (INCI), isohexadecane (INCI), triethylhexanoin (INCI), isotridecyl isononanoate (label name (INCI: Isotridecyl Isononanoate)), squalane (INCI), or the like.

The component (3) is different from the cross-linked silicone surfactant of the component (a) or the component (2) described above and is a compound having no polyether or polyglycerol structure in the molecular structure, and specific examples thereof include a (dimethicone/vinyl dimethicone) crosspolymer (INCI), a (dimethicone/phenyl vinyl dimethicone) crosspolymer (INCI), a (vinyl dimethicone/lauryl dimethicone) crosspolymer (INCI), and a (lauryl polydimethylsiloxyethyl dimethicone/bisvinyl dimethicone) crosspolymer (INCI).

Examples of commercially available compositions composed of a cross-linked organopolysiloxane and an oil agent that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z each manufactured by Shin-Etsu Chemical Co., Ltd.

### (4) Film-Forming Agent

The film-forming agent is blended mainly for the purpose of further maintaining the effects of the cosmetic. Although there is no particular limitation, a silicone composition is preferred from the perspective of imparting water repellency. More specifically, trimethylsiloxysilicic acid, an acrylic-silicone film agent, silicone-modified norbornene, silicone-modified pullulan, silicone-modified poly(vinyl alcohol), or the like can be used.

Examples of the film-forming agent of the silicone composition include trimethylsiloxysilicic acid (label name (INCI: Trimethylsiloxysilicate)), an (acrylates/dimethicone) copolymer (INCI), a (norbornene/tris(trimethylsiloxy)silylnorbornene) copolymer (INCI), and tri(trimethylsiloxy)silylpropylcarbamic acid pullulan (label name (INCI: Trimethylsiloxysilylcarbamoyl Pullulan)). The film-forming agent may be dissolved in advance in an oil agent that is liquid at room temperature and then blended in the cosmetic. The liquid oil agent may be a liquid silicone oil, hydrocarbon oil, ester oil, natural animal or vegetable oil, semi-synthetic oil, or fluorinated oil. Specific examples of commercially available silicone film-forming agents include KF-7312J, KP-545, KP-549, KP-543, NBN-30-ID, TSPL-30-ID, and TSPL-30-D5 each manufactured by Shin-Etsu Chemical Co., Ltd.

### (5) Aqueous Component

The aqueous component may be any aqueous component that can be blended in ordinary cosmetics. Specific examples thereof include water, ethanol (label name (INCI: Alcohol)), isopropanol (label name (INCI: Isopropyl Alcohol)), a lower alcohol preferably with 2 to 5 carbon atoms, and a sugar alcohol, such as sorbitol (INCI), maltose (INCI), or xylitol (INCI). Other examples thereof include a polyhydric alcohol, such as BG (label name (INCI: Butylene Glycol)), PG (label name (INCI: Propylene Glycol)), DPG (label name (INCI: Dipropylene Glycol)), pentylene glycol (INCI), 1,10-decanediol (INCI), octanediol (INCI), 1,2-hexanediol (INCI), erythritol (INCI), glycerol (INCI), diglycerol (INCI), or poly(ethylene glycol); and a humectant, such as glucose (INCI), glyceryl glucoside (INCI), betaine (INCI), chondroitin sulfate Na (label name (INCI: Sodium Chondroitin Sulfate)), PCA-Na (label name (INCI: Sodium PCA)), methyl gluceth-10 (INCI), methyl gluceth-20 (INCI), hyaluronic acid, egg-yolk lecithin, soybean lecithin, phosphatidylcholine, phosphatidyl ethanolamine, phosphatidyl serine, phosphatidyl glycerol, phosphatidyl inositol, or sphingophospholipid.

When the aqueous component (5) is blended, the blending amount thereof preferably ranges from 30% to 90% by mass, more preferably 50% to 88% by mass, still more preferably 60% to 85% by mass, particularly preferably 60% to 70% by mass, of the entire cosmetic. Less than 30% by mass may result in difficulty in feeling freshness. In particular, when a polyhydric alcohol is blended, the blending amount thereof preferably ranges from 1% to 50% by mass, more preferably 2% to 30% by mass, still more preferably 3% to 25% by mass, of the entire cosmetic.

### (6) Other Additive Agents

Examples of other additive agents include an oil-soluble gelling agent, a preservative/bactericide, an antiperspirant, a perfume, a salt, an antioxidant, a pH adjuster, a chelating agent, a refreshing agent, an anti-inflammatory agent, a skin-beautifying component (whitening agent, cell activator, skin roughness improving agent, blood circulation promoter, skin astringent, antiseborrheic agent, or the like), a vitamin, an amino acid, a nucleic acid, a hormone, and an inclusion compound.

### • Oil-Soluble Gelling Agent

The oil-soluble gelling agent may be a metallic soap, such as aluminum stearate, magnesium stearate, or zinc myristate; an amino acid derivative, such as lauroyl glutamic acid (label name (INCI: Lauroyl Glutamic Acid)) or α,γ-di-n-butylamine; a dextrin fatty acid ester, such as dextrin palmitate (label name (INCI: Dextrin Palmitate)), dextrin isostearate (label name (INCI: Dextrin Isostearate)), dextrin myristate (label name (INCI: Dextrin Myristate)), inulin stearate (label name (INCI: Stearoyl Inulin)), or dextrin (palmitate/ethylhexanoate) (label name (INCI: Dextrin Palmitate/Ethylhexanoate)); a sucrose fatty acid ester, such as sucrose palmitate or sucrose stearate; a fructo oligosaccharide fatty acid ester, such as fructo oligosaccharide stearate or fructo oligosaccharide 2-ethylhexanoate; a benzylidene derivative of sorbitol, such as monobenzylidene sorbitol or dibenzylidene sorbitol; an organic modified clay mineral, such as disteardimonium hectorite (INCI), stearalkonium hectorite (INCI), or hectorite; stearalkonium bentonite (INCI); or the like.

### • Preservative/Bactericide

The preservative/bactericide may be an alkyl p-hydroxybenzoate, benzoic acid, sodium benzoate, sorbic acid, potassium sorbate, phenoxyethanol, imidazolidinyl urea, salicylic acid, isopropylmethylphenol, phenol, p-chloro-m-cresol, hexachlorophene, benzalkonium chloride, chlorhexidine hydrochloride, trichlorocarbanilide, iodopropynyl butylcarbamate, polylysine, a photosensitive substance, silver, a plant extract, or the like.

### • Antiperspirant

The antiperspirant may be an aluminum hydroxyhalide, such as AL chlorohydrate, an aluminum halide, such as AL chloride, an allantoin aluminum salt, tannic acid, persimmon tannin, (AL/K) sulfate, zinc oxide, zinc p-phenolsulfonate, burnt alum, tetrachloro(Al/zirconium) hydrate, trichlorohydrex glycine (Al/zirconium), or the like. In particular, an aluminum hydroxyhalide, an aluminum halide, a complex or mixture thereof with a zirconyl oxyhalide or a zirconyl hydroxyhalide (for example, tetrachloro(Al/zirconium) hydrate or trichlorohydrex glycine (Al/zirconium)), or the like is preferred as a component that exhibits high effects.

### • Perfume

The perfume is a natural perfume or a synthetic perfume. The natural perfume is a plant perfume separated from a flower, leave, wood, pericarp, or the like; or an animal perfume, such as musk or civet. The synthetic perfume may be a hydrocarbon, such as monoterpene; an alcohol, such as an aliphatic alcohol or an aromatic alcohol; an aldehyde, such as a terpene aldehyde or an aromatic aldehyde; a ketone, such as an alicyclic ketone; an ester, such as a terpene ester; a lactone; a phenol; an oxide; a nitrogen-containing compound; an acetal; or the like.

### • Salts

The salt may be an inorganic salt, an organic acid salt, an amine salt, or an amino acid salt. The inorganic salt is, for example, a sodium salt, a potassium salt, a magnesium salt, a calcium salt, an aluminum salt, a zirconium salt, or a zinc salt of an inorganic acid, such as hydrochloric acid, sulfuric acid, carbonic acid, or nitric acid; the organic acid salt is, for example, a salt of an organic acid, such as acetic acid, dehydroacetic acid, citric acid, malic acid, succinic acid, ascorbic acid, or stearic acid; and the amine salt or an amino acid salt is, for example, a salt of an amine, such as triethanolamine, or a salt of an amino acid, such as glutamic acid. Furthermore, a salt, such as hyaluronic acid or chondroitin sulfate, an aluminum zirconium glycine complex, and an acid-alkali neutralized salt used in cosmetic formulations can also be used. Among these, Na chloride (label name (INCI: Sodium Chloride)) and Mg sulfate (label name (INCI: Magnesium Sulfate)) are preferred from the perspective of easy to handle and easy to blend in the formulation. When a salt is blended, the blending amount thereof preferably ranges from 0.1% to 3.0% by mass, more preferably 0.3% to 2.5% by mass, of the entire cosmetic.

### • Antioxidant

The antioxidant is, for example, but not limited to, a carotenoid, ascorbic acid or a salt thereof, ascorbyl stearate, tocopherol, tocopherol acetate, tocopherol, p-t-butylphenol, butylated hydroxyanisole, butylated hydroxytoluene, phytic acid, ferulic acid, thiotaurine, hypotaurine, sulfite, erythorbic acid or a salt thereof, chlorogenic acid, epicatechin, epigallocatechin, epigallocatechin gallate, apigenin, kaempferol, myricetin, quercetin, or the like. The antioxidant can be used alone or in combination of two or more types thereof.

### • pH Adjuster

The pH adjuster may be lactic acid, citric acid, glycolic acid, succinic acid, tartaric acid, dl-malic acid, potassium carbonate, sodium hydrogen carbonate, ammonium hydrogen carbonate, or the like.

### • Chelating Agent

The chelating agent may be alanine, sodium edetate, sodium polyphosphate, sodium metaphosphate, phosphoric acid, or the like.

### • Refreshing Agent

The refreshing agent may be L-menthol, camphor, menthyl lactate, or the like.

### • Anti-Inflammatory Agent

The anti-inflammatory agent may be allantoin, glycyrrhizic acid or a salt thereof, glycyrrhetinic acid, stearyl glycyrrhetinate, tranexamic acid, azulene, or the like.

### • Skin-Beautifying Component

The skin-beautifying component may be a whitening agent, such as a placenta extract, arbutin, glutathione, or a saxifrage extract, a wrinkle-improving agent, such as retinol or niacinamide, a cell activator, such as royal jelly, a photosensitive substance, a cholesterol derivative, or a calf blood extract; a skin roughness improving agent, a blood circulation promoter, such as nonivamide, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, or γ-oryzanol, a skin astringent, such as zinc oxide or tannic acid, an antiseborrheic agent, such as sulfur or thianthol, or the like.

### • Vitamin

The vitamin may be vitamin A, such as vitamin A oil, retinol, retinol acetate, or retinol palmitate, vitamin B, such as vitamin B2, such as riboflavin, riboflavin butyrate, or flavin adenine nucleotide, vitamin B6, such as pyridoxine hydrochloride, pyridoxine dioctanoate, or pyridoxine tripalmitate, vitamin B12 or a derivative thereof, or vitamin B15 or a derivative thereof, vitamin C, such as L-ascorbic acid, L-ascorbic acid dipalmitate, L-ascorbic acid-2-sodium sulfate, or L-ascorbic acid phosphoric diester dipotassium, vitamin D, such as ergocalciferol or cholecalciferol, vitamin E, such as α-tocopherol, β-tocopherol, γ-tocopherol, dl-α-tocopherol acetate, dl-α-tocopherol nicotinate, or dl-α-tocopherol succinate; nicotinic acid, such as nicotinic acid, benzyl nicotinate, or nicotinamide, vitamin H, vitamin P, a pantothenic acid, such as calcium pantothenate, D-pantothenyl alcohol, pantothenyl ethyl ether, or acetylpantothenyl ethyl ether, biotin, or the like.

### • Amino Acid

The amino acid may be glycine, valine, leucine, isoleucine, serine, threonine, phenylalanine, arginine, lysine, aspartic acid, glutamic acid, cystine, cysteine, methionine, tryptophan, or the like.

### • Nucleic Acid

The nucleic acid may be deoxyribonucleic acid or the like.

### • Hormone

The hormone may be estradiol, ethenyl estradiol, or the like.

### • Inclusion Compound

The inclusion compound may be cyclodextrin or the like.

### EXAMPLES

Although the present invention will be more specifically described with reference to Production Examples, Comparative Production Examples, and Examples and Comparative Examples of cosmetics, the present invention is not limited to these Examples. Unless otherwise specified, "%" in the composition described below means "% by mass", the mass percentage of each component is based on the total mass (100% by mass) in each example, and the blending amount is the blending amount of the blended product described.

### [Production Example 1]

A (dimethicone/polyglycerin-3) crosspolymer produced by reacting a linear dimethyl hydrogen polysiloxane with a glycerol derivative having two or more alkenyl groups per molecule in the presence of a hydrosilylation reaction catalyst was mixed with caprylyl methicone (label name) (kinematic viscosity: 3 mm²/s, KF-4418 manufactured by Shin-Etsu Chemical Co., Ltd.) using a three-roll mill to prepare a gel composition (silicone gel) with a component (a) concentration of 30%.

### [Production Example 2]

A (dimethicone/polyglycerin-3) crosspolymer produced by reacting a linear dimethyl hydrogen polysiloxane with a glycerol derivative having two or more alkenyl groups per molecule in the presence of a hydrosilylation reaction catalyst was mixed with caprylyl methicone (label name) (kinematic viscosity: 3 mm²/s, KF-4418 manufactured by Shin-Etsu Chemical Co., Ltd.) using a three-roll mill to prepare a gel composition (silicone gel) with a component (a) concentration of 15%.

### [Production Example 3]

A (dimethicone/polyglycerin-3) crosspolymer produced by reacting a linear dimethyl hydrogen polysiloxane with a glycerol derivative having two or more alkenyl groups per molecule in the presence of a hydrosilylation reaction catalyst was mixed with caprylyl methicone (label name) (kinematic viscosity: 3 mm²/s, KF-4418 manufactured by Shin-Etsu Chemical Co., Ltd.) using a three-roll mill to prepare a gel composition (silicone gel) with a component (a) concentration of 60%.

### [Production Example 4]

A (dimethicone/polyglycerin-3) crosspolymer produced by reacting a linear dimethyl hydrogen polysiloxane with a glycerol derivative having two or more alkenyl groups per molecule in the presence of a hydrosilylation reaction catalyst was mixed with ethyl methicone (label name) (kinematic viscosity: 2.2 mm²/s, KF-4422 manufactured by Shin-Etsu Chemical Co., Ltd.) using a three-roll mill to prepare a gel composition (silicone gel) with a component (a) concentration of 30%.

### [Production Example 5]

A (dimethicone/polyglycerin-3) crosspolymer produced by reacting a linear dimethyl hydrogen polysiloxane with a glycerol derivative having two or more alkenyl groups per molecule in the presence of a hydrosilylation reaction catalyst was mixed with methyl trimethicone (label name) (kinematic viscosity: 1.5 mm²/s, TMF-1.5 manufactured by Shin-Etsu Chemical Co., Ltd.) using a three-roll mill to prepare a gel composition (silicone gel) with a component (a) concentration of 30%.

### [Comparative Production Example 1]

A (dimethicone/polyglycerin-3) crosspolymer was mixed with a dimethicone (label name) with a kinematic viscosity of 6 mm²/s at 25°C (KF-96A-6cs manufactured by Shin-Etsu Chemical Co., Ltd.) using a three-roll mill to prepare a gel composition (silicone gel) with a component (a) concentration of 30%.

The advantages of the present invention will be described using Examples and Comparative Examples relating to cosmetics. Production Examples and Comparative Production Examples represent gel compositions (silicone gels) produced in the respective Examples.

### [Examples 1 to 7, Comparative Examples 1 to 3]

A W/O makeup base (water-in-oil emulsion) was prepared according to the formulation shown in Tables 1 and 2.

### (Production Method)

| | |
|---|---|
| Preparation of oil phase: | The components 1 to 7 were uniformly mixed. |
| Preparation of aqueous phase: | The components 8 to 10 were uniformly mixed. |

The aqueous phase was gently added to the oil phase and was emulsified to prepare a W/O makeup base.

The W/O makeup base was evaluated in terms of (1) emulsifiability, (2) usability (no stickiness), and (3) applicability (spreadability and adherence of the cosmetic) according to the following evaluation criteria. The results are also shown in Table 1.

### [Emulsifiability]

The state at the time of transferring the aqueous phase to the oil phase and the state at the time of emulsification were observed and evaluated according to the following criteria using the results of Example 1 as a standard.
A: Both transfer and emulsification are possible (standard).
B: Although transfer and emulsification are possible, 10% or more and less than 30% of time is additionally required for transfer as compared with the standard.
C: Although transfer and emulsification are possible, 30% or more and less than 40% of the time is additionally required for transfer as compared with the standard.
D: Although transfer is possible, the aqueous phase is discharged during emulsification.
E: Transfer is not possible even if 40% or more of the time is added as compared with the standard.

"C" or higher (A, B, and C) was regarded as acceptable.

### [Property Evaluation]

The resulting cosmetics were evaluated in terms of the usability (no stickiness) and applicability by 10 expert panelists according to the evaluation criteria shown below. The results were evaluated according to the following determination criteria based on the average value of the 10 panelists. However, a cosmetic that could not be transferred or emulsified could not be evaluated, and was indicated by "-".

### [Evaluation Criteria]

| | |
|---|---|
| 5 points: | very good |
| 4 points: | good |
| 3 points: | fair |
| 2 points: | slightly poor |
| 1 point: | poor |

### [Determination Criteria Based on Average Score]

A: average score of 4.5 or more
B: average score of 3.5 or more and less than 4.5
C: average score of 2.5 or more and less than 3.5
D: average score of 1.5 or more and less than 2.5
E: average score of less than 1.5

"C" or higher (A, B and C) was regarded as acceptable.

**[Table 1]**

| Composition (%) | | Example | | | Comparative Example |
|---|---|---|---|---|---|
| | | 1 | 2 | 3 | 1 |
| 1 | Production Example 1 | 4 | 4 | 4 | |
| 2 | Comparative Production Example 1 | | | | 4 |
| 3 | KF-4418 (Note 1) | 16 | 15.5 | 13.2 | 16 |
| 4 | Dimethicone 6 cs | | | 2.8 | |
| 5 | Dimethicone 100 cs | | 0.5 | | |
| 8 | Na chloride | 0.5 | 0.5 | 0.5 | 0.5 |
| 9 | BG | 5 | 5 | 5 | 5 |
| 10 | Water | 74.5 | 74.5 | 74.5 | 74.5 |
| Total | | 100.0 | 100.0 | 100.0 | 100.0 |
| Amount of component (a) (entire cosmetic) | | 1.2 | 1.2 | 1.2 | 1.2 |
| Amount of component (b) (entire cosmetic) | | 18.8 | 18.3 | 16 | 16 |
| Emulsifiability | | A | A | A | A |
| Usability | | A | B | C | D |
| Applicability | | A | A | A | B |

| | | | | | |
|---|---|---|---|---|---|
| (Note 1) Caprylyl methicone manufactured by Shin-Etsu Chemical Co., Ltd.: component (b) | | | | | |

**[Table 2]**

| Composition (%) | | Example | | | | Comparative Example | |
|---|---|---|---|---|---|---|---|
| | | 4 | 5 | 6 | 7 | 2 | 3 |
| 1 | Production Example 1 | 4 | 4 | 4 | 4 | | |
| 2 | Comparative Production Example 1 | | | | | 4 | 4 |
| 3 | KF-4418 (Note 1) | 15 | 6.5 | 6 | 6 | 6.5 | 6 |
| 6 | (c) Ethylhexyl methoxycinnamate | | 9.5 | 9.5 | 9 | 9.5 | 9 |
| 7 | (d) KTP-09W (Note 2) | 1 | | | 1 | | 1 |
| 8 | Na chloride | 0.5 | 0.5 | 0.5 | 0.5 | 0.5 | 0.5 |
| 9 | BG | 5 | 5 | 5 | 5 | 5 | 5 |
| 10 | Water | 74.5 | 74.5 | 74.5 | 74.5 | 74.5 | 74.5 |
| Total | | 100.0 | 100.0 | 100.0 | 100.0 | 100.0 | 100.0 |
| Amount of component (a) (entire cosmetic) | | 1.2 | 1.2 | 1.2 | 1.2 | 1.2 | 1.2 |
| Amount of component (b) (entire cosmetic) | | 17.8 | 9.3 | 8.8 | 8.8 | 6.5 | 6.0 |
| Amount of component (c) (entire cosmetic) | | 0 | 9.5 | 9.5 | 9 | 9.5 | 9 |
| Amount of component (d) (entire cosmetic) | | 1 | 0 | 0 | 1 | 0 | 1 |
| Emulsifiability | | A | A | B | B | E | E |
| Usability | | B | C | C | C | - | - |
| Applicability | | A | A | A | B | - | - |

| | | | | | | | |
|---|---|---|---|---|---|---|---|
| (Note 1) Caprylyl methicone manufactured by Shin-Etsu Chemical Co., Ltd.: component (b) (Note 2) KF-9909-treated color inorganic pigments, W: white, manufactured by Shin-Etsu Chemical Co., Ltd. | | | | | | | |

As is clear from the above results, it was confirmed that the W/O makeup bases of Examples 1 to 5 were easily emulsified and had high usability and applicability. On the other hand, Comparative Example 1 could be emulsified but had poor usability, and Comparative Examples 2 and 3 were difficult to formulate into a cosmetic. Examples 1 and 2 were particularly easy to emulsify and had high usability and applicability. Comparative Example 1, although containing the components (a) and (b) of the present invention, used an oil agent other than the component (b) of the present invention to swell the component (a) and was not a silicone gel in which the following component (a) was swollen in advance with the component (b), and exhibited poorer usability than Example 3. Furthermore, although Examples 4 to 6 could be stably emulsified even when the component (c) or (d) was blended, Comparative Examples 2 and 3 in which a silicone gel of the present invention was not blended could not be emulsified.

The gel compositions (silicone gels) of Production Examples 1 to 5 were used to prepare the following cosmetics. As shown below, an effect equivalent to that of Production Example 1 could be produced.

### [Example 8] Water-in-Oil Lipstick

| Composition | | % |
|---|---|---|
| 1. | KF-56A (Note 1) | 11.2 |
| 2. | Production Example 1 (component a: 30%, component b: 70%) | 3 |
| 3. | KSG-18A (Note 2) | 1 |
| 4. | KF-6048 (Note 3) | 1 |
| 5. | KSP-441 (Note 4) | 3 |
| 6. | Ceresin | 5 |
| 7. | Inulin stearate | 3 |
| 8. | Hydrogenated polyisobutene | 5 |
| 9. | (Caprylic/capric) triglyceride | 3 |
| 10. | Neopentyl glycol dicaprate | 2 |
| 11. | Mineral oil | 3 |
| 12. | Sorbitan sesquiisostearate | 0.5 |
| 13. | Red 201 | 0.4 |
| 14. | Red 202 | 0.4 |
| 15. | Yellow 4 | 1.6 |
| 16. | KTP-09W (Note 5) | 3 |
| 17. | KTP-09R (Note 5) | 0.7 |
| 18. | KTP-09B (Note 5) | 0.2 |
| 19. | KP-574 (Note 6) treated talc | 0.7 |
| 20. | Glycerol | 10 |
| 21. | Pentylene glycol | 3 |
| 22. | Ethylhexyl glycerol | 0.1 |
| 23. | Purified water | 40 |
| Total | | 100.0 |

| | |
|---|---|
| (Note 1) | Diphenylsiloxyphenyl trimethicone manufactured by Shin-Etsu Chemical Co., Ltd. |
| (Note 2) | A mixture of diphenylsiloxyphenyl trimethicone 80% to 90% + (dimethicone/phenyl vinyl dimethicone) crosspolymer 10% to 20% manufactured by Shin-Etsu Chemical Co., Ltd. |
| (Note 3) | Cetyl PEG/PPG-10/1 dimethicone manufactured by Shin-Etsu Chemical Co., Ltd. |
| (Note 4) | Polysilicone-22 manufactured by Shin-Etsu Chemical Co., Ltd. |
| (Note 5) | KF-9909-treated color inorganic pigments W: white, R: red, Y: yellow, B: black each manufactured by Shin-Etsu Chemical Co., Ltd. |
| (Note 6) | An (acrylates/tridecyl acrylate/triethoxysilylpropyl methacrylate/dimethicone methacrylate) copolymer manufactured by Shin-Etsu Chemical Co., Ltd. |

### (Production Method)

A: The components 10 to 19 were dispersed with a three-roll mill.
B: The components 1 to 9 were uniformly mixed at 95°C.
C: The components 20 to 23 were uniformly mixed at 85°C.
D: The dispersion prepared in A was added to the mixture prepared in B, and the mixture prepared in C was added thereto at 85°C and was emulsified. The emulsion was poured into a stick container and was cooled to prepare a water-in-oil lipstick.

The water-in-oil lipstick was a cosmetic with high usability, applicability, and emulsion stability.

### [Example 9] Water-in-Oil Sunscreen Cream

| Composition | | % |
|---|---|---|
| 1. | Production Example 5 (component a: 30%, component b: 70%) | 3 |
| 2. | KSG-41A (Note 1) | 3 |
| 3. | KF-6105 (Note 2) | 1.5 |
| 4. | KF-56A (Note 3) | 11 |
| 5. | Ethylhexyl methoxycinnamate | 7 |
| 6. | KSP-100 (Note 4) | 2 |
| 7. | Xanthan gum | 0.3 |
| 8. | Dipropylene glycol | 5 |
| 9. | Glycerol | 3 |
| 10. | Methylparaben | 0.1 |
| 11. | Sodium citrate | 0.2 |
| 12. | Sodium chloride | 0.5 |
| 13. | Water | 63.4 |
| Total | | 100.0 |

| | |
|---|---|
| (Note 1) | A mixture of mineral oil 70% to 80% + (vinyl dimethicone/lauryl dimethicone) crosspolymer 20% to 30% manufactured by Shin-Etsu Chemical Co., Ltd. |
| (Note 2) | Lauryl polyglyceryl-3 polydimethylsiloxyethyl dimethicone manufactured by Shin-Etsu Chemical Co., Ltd. |
| (Note 3) | Diphenylsiloxyphenyl trimethicone manufactured by Shin-Etsu Chemical Co., Ltd. |
| (Note 4) | A (vinyl dimethicone/methicone silsesquioxane) crosspolymer manufactured by Shin-Etsu Chemical Co., Ltd. |

### (Production Method)

A: The components 1 to 6 were uniformly mixed.
B: The components 7 to 13 were uniformly mixed.
C: The mixture prepared in B was added to the mixture prepared in A and was emulsified to prepare a water-in-oil sunscreen cream.

The water-in-oil sunscreen cream was a cosmetic with high usability, high applicability, high emulsion stability, and a good soft focus effect.

### [Example 10] W/O Cosmetic Stick

| Composition | | % |
|---|---|---|
| 1. | Production Example 1 (component a: 30%, component b: 70%) | 5 |
| 2. | KSG-42A (Note 1) | 5 |
| 3. | KF-6105 (Note 2) | 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | BG | 7 |
| 9. | PCA-Na | 2 |
| 10. | Diglycerol | 3 |
| 11. | Na citrate | 0.2 |
| 12. | Mg sulfate | 1 |
| 13. | Water | 55.8 |
| Total | | 100.0 |

| | |
|---|---|
| (Note 1) | A mixture of isododecane 75% to 85% + (vinyl dimethicone/lauryl dimethicone) crosspolymer 15% to 25% manufactured by Shin-Etsu Chemical Co., Ltd. |
| (Note 2) | Lauryl polyglyceryl-3 polydimethylsiloxyethyl dimethicone manufactured by Shin-Etsu Chemical Co., Ltd. |
| (Note 3) | Diphenylsiloxyphenyl trimethicone manufactured by Shin-Etsu Chemical Co., Ltd. |

### (Production Method)

A: The components 1 to 7 were uniformly mixed at 90°C.
B: The components 8 to 13 were uniformly mixed at 85°C.
C: The mixture prepared in B was added to the mixture prepared in A, was emulsified, and was charged into a stick container to prepare a W/O cosmetic stick.

The W/O cosmetic stick was a cosmetic with high usability, high applicability, high emulsion stability, and a good soft focus effect.

### [Example 11] Balm

| Composition | | % |
|---|---|---|
| 1. | Vaseline | 27 |
| 2. | Squalane | 25.8 |
| 3. | KSP-101 (Note 1) | 10 |
| 4. | KMP-590 (Note 2) | 6 |
| 5. | Production Example 1 (component a: 30%, component b: 70%) | 5 |
| 6. | KSG-43 (Note 3) | 3 |
| 7. | Dimethicone (6 cs) | 4 |
| 8. | KF-56A (Note 4) | 7 |
| 9. | KP-561P (Note 5) | 2 |
| 10. | KF-6038 (Note 6) | 0.2 |
| 11. | Dextrin palmitate | 10 |
| Total | | 100.0 |

| | |
|---|---|
| (Note 1) | A (vinyl dimethicone/methicone silsesquioxane) crosspolymer manufactured by Shin-Etsu Chemical Co., Ltd. |
| (Note 2) | Polymethylsilsesquioxane manufactured by Shin-Etsu Chemical Co., Ltd. |
| (Note 3) | A mixture of triethylhexanoin 65% to 75% + (vinyl dimethicone/lauryl dimethicone) crosspolymer 25% to 35% manufactured by Shin-Etsu Chemical Co., Ltd. |
| (Note 4) | Diphenylsiloxyphenyl trimethicone manufactured by Shin-Etsu Chemical Co., Ltd. |
| (Note 5) | An (acrylates/stearyl acrylate/dimethicone methacrylate) copolymer manufactured by Shin-Etsu Chemical Co., Ltd. |
| (Note 6) | Lauryl PEG-9 polydimethylsiloxyethyl dimethicone manufactured by Shin-Etsu Chemical Co., Ltd. |

### (Production Method)

A: The components 1 to 11 were uniformly mixed at 90°C and were cooled to prepare a balm.

The balm was a cosmetic with high usability, high applicability, high stability, and a good soft focus effect.

### [Example 12] O/W Sunscreen Cream

| Composition | | % |
|---|---|---|
| 1. | Hydroxyethyl cellulose | 0.1 |
| 2. | Ethanol | 10 |
| 3. | BG | 6 |
| 4. | Allantoin | 0.1 |
| 5. | SIMULGEL EG (Note 1) | 2.5 |
| 6. | Water | 59.25 |
| 7. | KF-56A (Note 2) | 3 |
| 8. | Production Example 1 (component a: 30%, component b: 70%) | 1 |
| 9. | Cetanol | 2 |
| 10. | Ethylhexyl methoxycinnamate | 7.5 |
| 11. | Diethylamino hydroxybenzoyl hexyl benzoate | 2 |
| 12. | Ethylhexyl salicylate | 5 |
| 13. | Polyoxyethylene (60) hydrogenated castor oil | 1 |
| 14. | KF-6011 (Note 3) | 0.5 |
| 15. | Tocopherol | 0.05 |
| Total | | 100.0 |

| | |
|---|---|
| (Note 1) | A sodium acrylate-sodium acryloyldimethyltaurate copolymer composition manufactured by SEPPIC |
| (Note 2) | Diphenylsiloxyphenyl trimethicone manufactured by Shin-Etsu Chemical Co., Ltd. |
| (Note 3) | PEG-11 methyl ether dimethicone manufactured by Shin-Etsu Chemical Co., Ltd. |

### (Production Method)

A: The components 1 to 6 were heated to 85°C and were uniformly mixed.
B: The components 7 to 15 were heated to 85°C and were uniformly mixed.
C: The mixture prepared in B was added to the mixture prepared in A, was emulsified at 85°C, and was then gradually cooled with stirring to prepare an O/W sunscreen cream.

The O/W sunscreen cream was a cosmetic with high usability, high applicability, high stability, and a good soft focus effect.

### [Example 13] Sunscreen Gel

| Composition | | % |
|---|---|---|
| 1. | KSG-42A (Note 1) | 30 |
| 2. | KSG-042Z (Note 2) | 25.5 |
| 3. | Production Example 4 (component a: 30%, component b: 70%) | 5 |
| 4. | KF-4422 (Note 3) (component b) | 2.5 |
| 5. | KF-56A (Note 4) | 5 |
| 6. | Octocrylene | 2 |
| 7. | Homosalate | 10 |
| 8. | Ethylhexyl salicylate | 5 |
| 9. | Bisethylhexyloxyphenol methoxyphenyl triazine | 3 |
| 10. | KSP-441 (Note 5) | 10 |
| 11. | KMP-592 (Note 6) | 2 |
| Total | | 100.0 |

| | |
|---|---|
| (Note 1) | A mixture of isododecane 75% to 85% + (vinyl dimethicone/lauryl dimethicone) crosspolymer 15% to 25% manufactured by Shin-Etsu Chemical Co., Ltd. |
| (Note 2) | A mixture of isododecane 75% to 85% + (lauryl polydimethylsiloxyethyl dimethicone/bisvinyl dimethicone) crosspolymer 15% to 25% manufactured by Shin-Etsu Chemical Co., Ltd. |
| (Note 3) | Ethyl methicone manufactured by Shin-Etsu Chemical Co., Ltd. |
| (Note 4) | Diphenylsiloxyphenyl trimethicone manufactured by Shin-Etsu Chemical Co., Ltd. |
| (Note 5) | Polysilicone-22 manufactured by Shin-Etsu Chemical Co., Ltd. |
| (Note 6) | (Methyl/phenyl) polysilsesquioxane manufactured by Shin-Etsu Chemical Co., Ltd. |

### (Production Method)

A: The components 1 to 11 were uniformly mixed to prepare a sunscreen gel.

The sunscreen gel was a cosmetic with high usability, high applicability, high stability, and a good soft focus effect.

### [Example 14] Stick Concealer

| Composition | | % |
|---|---|---|
| 1. | Production Example 1 (component a: 30%, component b: 70%) | 6 |
| 2. | KSP-411 (Note 1) | 10 |
| 3. | KMP-592 (Note 2) | 16 |
| 4. | Dimethicone (6 cs) | 2 |
| 5. | KF-4418 (Note 3) (component b) | 30 |
| 6. | Triethylhexanoin | 20 |
| 7. | Microcrystalline wax | 6 |
| 8. | Ceresin | 10 |
| Total | | 100.0 |

| | |
|---|---|
| (Note 1) | A polysilicone-1 crosspolymer manufactured by Shin-Etsu Chemical Co., Ltd. |
| (Note 2) | (Methyl/phenyl) polysilsesquioxane manufactured by Shin-Etsu Chemical Co., Ltd. |
| (Note 3) | Caprylyl methicone manufactured by Shin-Etsu Chemical Co., Ltd. |

### (Production Method)

A: The components 1 to 8 were uniformly mixed at 95°C and were charged into a stick container to prepare a stick concealer.

The stick concealer was a cosmetic with high usability, high applicability, high stability, and a good soft focus effect.

### [Example 15] Lipstick

| Composition | | % |
|---|---|---|
| 1. | Production Example 1 (component a: 30%, component b: 70%) | 5 |
| 2. | Polyethylene | 7 |
| 3. | Microcrystalline wax | 3 |
| 4. | KP-561P (Note 1) | 10 |
| 5. | Diisostearyl malate | 13 |
| 6. | Neopentyl glycol diethylhexanoate | 15 |
| 7. | Neopentyl glycol dicaprate | 7 |
| 8. | Hydrogenated polyisobutene | Balance |
| 9. | KF-54HV (Note 2) | 8 |
| 10. | Pearl pigment | 5 |
| 11. | Mica | 0.3 |
| 12. | Red 202 | 0.1 |
| 13. | Yellow 4 | 0.3 |
| 14. | KP-574 (Note 3) treated titanium dioxide | 0.8 |
| 15. | KP-574 (Note 3) treated black iron oxide | 0.06 |
| 16. | Red 201 | 0.04 |
| 17. | KP-574 (Note 3) treated red oxide | 0.2 |
| 18. | Triisostearate polyglyceryl-2 | 1.2 |
| Total | | 100.0 |

| | |
|---|---|
| (Note 1) | An (acrylates/stearyl acrylate/dimethicone methacrylate) copolymer manufactured by Shin-Etsu Chemical Co., Ltd. |
| (Note 2) | Diphenyl dimethicone manufactured by Shin-Etsu Chemical Co., Ltd. |
| (Note 3) | An (acrylates/tridecyl acrylate/triethoxysilylpropyl methacrylate/dimethicone methacrylate) copolymer manufactured by Shin-Etsu Chemical Co., Ltd. |

### (Production Method)

A: The components 11 to 18 were dispersed using a roll.
B: The components 1 to 9 were uniformly mixed at 95°C.
C: The mixture prepared in A and 10 were added to the mixture prepared in B and were cooled to prepare a lipstick.

The lipstick was a cosmetic with high usability, high applicability, high stability, and a good soft focus effect.

### [Example 16] Mascara

| Composition | | % |
|---|---|---|
| 1. | Production Example 1 (component a: 30%, component b: 70%) | 10 |
| 2. | TSPL-30-ID (Note 1) (component b: 70%) | 10 |
| 3. | Isododecane (component b) | 41 |
| 4. | Dextrin palmitate | 2 |
| 5. | Ceresin | 7 |
| 6. | Microcrystalline wax | 7 |
| 7. | Disteardimonium hectorite | 5 |
| 8. | Alkylsilane-treated black iron oxide (Note 2) | 5 |
| 9. | Alkylsilane-treated talc (Note 2) | 5 |
| 10. | KMP-590 (Note 3) | 5 |
| 11. | KF-6017 (Note 4) | 1.2 |
| 12. | Propylene carbonate | 1.6 |
| 13. | Phenoxvethanol | 0.2 |
| Total | | 100.0 |

| | |
|---|---|
| (Note 1) | Tri(trimethylsiloxy)silylpropylcarbamic acid pullulan manufactured by Shin-Etsu Chemical Co., Ltd. |
| (Note 2) | manufactured by Shin-Etsu Chemical Co., Ltd., treated with AES-3083 |
| (Note 3) | Polymethylsilsesquioxane manufactured by Shin-Etsu Chemical Co., Ltd. |
| (Note 4) | PEG-10 dimethicone manufactured by Shin-Etsu Chemical Co., Ltd. |

### (Production Method)

A: The components 1 to 7 were uniformly mixed at 90°C.
B: The components 8 to 13 were added to the mixture prepared in A and were uniformly mixed to prepare a mascara.

The mascara was a cosmetic with high usability, applicability, and stability.

### [Example 17] Hair Oil

| Composition | | % |
|---|---|---|
| 1. | Production Example 1 (component a: 30%, component b: 70%) | 3 |
| 2. | KF-56A (Note 1) | 7 |
| 3. | Diethylhexyl succinate | 10 |
| 4. | X-21-5613 (note 2) | 1.5 |
| 5. | Tocopherol | 0.1 |
| 6. | Perfume | 0.1 |
| 7. | Light liquid isoparaffin | Balance |
| Total | | 100.0 |

| | |
|---|---|
| (Note 1) | Diphenylsiloxyphenyl trimethicone manufactured by Shin-Etsu Chemical Co., Ltd. |
| (Note 2) | Dimethiconol manufactured by Shin-Etsu Chemical Co., Ltd. |

### (Production Method)

A: The components 1 to 7 were uniformly mixed, hair oil.

The hair oil was a cosmetic with high usability, high applicability, high stability, and a good soft focus effect.

### [Example 18] Hair Treatment

| Composition | | % |
|---|---|---|
| 1. | Production Example 1 (component a: 30%, component b: 70%) | 1 |
| 2. | Cetanol | 2 |
| 3. | Cetyl octanoate | 3 |
| 4. | Butyl p-hydroxybenzoate | 0.1 |
| 5. | KF-56A (note 1) | 1 |
| 6. | Behentrimonium chloride | 1 |
| 7. | Propylene glycol | 5 |
| 8. | Hydroxyethyl cellulose | 0.1 |
| 9. | Water | Balance |
| 10. | X-52-2328 (Note 2) | 4 |
| 11. | Perfume | q.s. |
| Total | | 100.0 |

| | |
|---|---|
| (Note 1) | Diphenylsiloxyphenyl trimethicone manufactured by Shin-Etsu Chemical Co., Ltd. |
| (Note 2) | Aminopropyl dimethicone manufactured by Shin-Etsu Chemical Co., Ltd. |

### (Production Method)

A: The components 6 to 9 were heated to 70°C and were uniformly mixed.
B: The components 1 to 5 were heated to 70°C and were uniformly mixed.
C: The mixture prepared in B was added to the mixture prepared in A, was emulsified, and was gradually cooled, and the components 10 and 11 were then added thereto to prepare a hair treatment.

The hair treatment was a cosmetic with high usability, high applicability, high stability, and a good soft focus effect.

### [Example 19] W/O Cream

| Composition | | % |
|---|---|---|
| 1. | KSG-710 (Note 1) | 3 |
| 2. | Production Example 1 (component a: 30%, component b: 70%) | 3 |
| 3. | KSG-43 (Note 2) | 2 |
| 4. | Octyldodecyl myristate | 13.5 |
| 5. | Macadamia nut oil | 5 |
| 6. | KF-6038 (Note 3) | 1 |
| 7. | KSP-100 (Note 4) | 2 |
| 8. | KMP-590 (Note 5) | 1 |
| 9. | Sodium citrate | 0.2 |
| 10. | DPG | 8 |
| 11. | Diglycerol | 3 |
| 12. | Water | 58.3 |
| Total | | 100.0 |

| | |
|---|---|
| (Note 1) | A mixture of dimethicone 70% to 80% + (dimethicone/polyglycerin-3) crosspolymer 20% to 30% manufactured by Shin-Etsu Chemical Co., Ltd. |
| (Note 2) | A mixture of triethylhexanoin 65% to 75% + (vinyl dimethicone/lauryl dimethicone) crosspolymer 25% to 35% manufactured by Shin-Etsu Chemical Co., Ltd. |
| (Note 3) | Lauryl PEG-9 polydimethylsiloxyethyl dimethicone manufactured by Shin-Etsu Chemical Co., Ltd. |
| (Note 4) | A (vinyl dimethicone/methicone silsesquioxane) crosspolymer manufactured by Shin-Etsu Chemical Co., Ltd. |
| (Note 5) | Polymethylsilsesquioxane manufactured by Shin-Etsu Chemical Co., Ltd. |

### (Production Method)

A: The components 1 to 8 were uniformly mixed.
B: The components 9 to 12 were uniformly mixed.
C: While stirring, the mixture prepared in B was gradually added to the mixture prepared in A and was emulsified to prepare a W/O cream.

The W/O cream was a cosmetic with high usability, high applicability, high emulsion stability, and a good soft focus effect.

### [Example 20] W/O Cream Foundation

| Composition | | % |
|---|---|---|
| 1. | KSG-19 (Note 1) | 2 |
| 2. | Production Example 1 (component a: 30%, component b: 70%) | 2 |
| 3. | Mineral oil | 2 |
| 4. | Triethylhexanoin | 5 |
| 5. | Isotridecyl isononanoate | 9 |
| 6. | KF-6038 (Note 2) | 1.5 |
| 7. | KMP-591 (Note 3) | 1 |
| 8. | KSP-100 (Note 4) | 2 |
| 9. | KTP-09W, R, Y, B (Note 5) | 20 |
| 10. | KF-6115 (Note 6) | 0.5 |
| 11. | BG | 5 |
| 12. | Sodium citrate | 1 |
| 13. | Magnesium sulfate | 1 |
| 14. | Water | 48 |
| Total | | 100.0 |

| | |
|---|---|
| (Note 1) | A mixture of dimethicone (6 cs) 80% to 90% + (dimethicone/vinyl dimethicone) crosspolymer 10% to 20% manufactured by Shin-Etsu Chemical Co., Ltd. |
| (Note 2) | Lauryl PEG-9 polydimethylsiloxyethyl dimethicone manufactured by Shin-Etsu Chemical Co., Ltd. |
| (Note 3) | Polymethylsilsesquioxane manufactured by Shin-Etsu Chemical Co., Ltd. |
| (Note 4) | A (vinyl dimethicone/methicone silsesquioxane) crosspolymer manufactured by Shin-Etsu Chemical Co., Ltd. |
| (Note 5) | KF-9909-treated color inorganic pigments W: white, R: red, Y: yellow, B: black each manufactured by Shin-Etsu Chemical Co., Ltd. |
| (Note 6) | Lauryl polyglyceryl-3 polydimethylsiloxyethyl dimethicone manufactured by Shin-Etsu Chemical Co., Ltd. |

### (Production Method)

A: The components 1 to 6 were uniformly mixed, and the components 7 to 10 were added thereto and were homogenized.
B: The components 11 to 14 were uniformly mixed.
C: While stirring, the mixture prepared in B was gradually added to the mixture prepared in A and was emulsified to prepare a W/O cream foundation.

The W/O cream foundation was a cosmetic with high usability, high applicability, high emulsion stability, and a good soft focus effect.

### [Example 21] W/O Water Break Cream

| Composition | | % |
|---|---|---|
| 1. | Production Example 1 (component a: 30%, component b: 70%) | 3 |
| 2. | KF-6048 (Note 1) | 0.2 |
| 3. | KSG-44 (Note 2) | 1 |
| 4. | Squalane | 10.8 |
| 5. | BG | 8 |
| 6. | Ethanol | 5 |
| 7. | Sodium citrate | 0.2 |
| 8. | Sodium chloride | 0.5 |
| 9. | Water | 71.3 |
| Total | | 100.0 |

| | |
|---|---|
| (Note 1) | Cetyl PEG/PPG-10/1 dimethicone manufactured by Shin-Etsu Chemical Co., Ltd. |
| (Note 2) | A mixture of squalane 65% to 75% + (vinyl dimethicone/lauryl dimethicone) crosspolymer 25% to 35% manufactured by Shin-Etsu Chemical Co., Ltd. |

### (Production Method)

A: The components 1 to 4 were uniformly mixed.
B: The components 5 to 9 were uniformly mixed.
C: The mixture prepared in B was added to the mixture prepared in A and was emulsified to prepare a W/O water break cream.

The W/O water break cream was a cosmetic with high usability, high applicability, high emulsion stability, and a good soft focus effect.

### [Example 22] W/O Cream Foundation

| Composition | | % |
|---|---|---|
| 1. | KSG-270 (Note 1) | 0.5 |
| 2. | Production Example 1 (component a: 30%, component b: 70%) | 3 |
| 3. | KF-6028 (Note 2) | 3 |
| 4. | KSG-18A (Note 3) | 6 |
| 5. | Organic modified clay mineral | 1.2 |
| 6. | Cyclopentasiloxane (component b) | 20 |
| 7. | Ethylhexyl methoxycinnamate | 7.5 |
| 8. | KF-7312J (Note 4) | 2 |
| 9. | KMP-591 (Note 5) | 2 |
| 10. | Ethylhexyl palmitate | 7 |
| 11. | KP-578 (Note 6) | 0.2 |
| 12. | KTP-09W, R, Y, B (Note 7) | 10 |
| 13. | BG | 5 |
| 14. | Methylparaben | 0.15 |
| 15. | Sodium citrate | 0.2 |
| 16. | Sodium chloride | 0.5 |
| 17. | Water | Balance |
| Total | | 100.0 |

| | |
|---|---|
| (Note 1) | A mixture of diphenylsiloxyphenyl trimethicone 75% to 85% + (dimethicone/(PEG-10/15)) crosspolymer 15% to 25% manufactured by Shin-Etsu Chemical Co., Ltd. |
| (Note 2) | PEG-9 polydimethylsiloxyethyl dimethicone manufactured by Shin-Etsu Chemical Co., Ltd. |
| (Note 3) | A mixture of diphenylsiloxyphenyl trimethicone 80% to 90% + (dimethicone/phenyl vinyl dimethicone) crosspolymer 10% to 20% manufactured by Shin-Etsu Chemical Co., Ltd. |
| (Note 4) | A solution of 50% trimethylsiloxysilicic acid in cyclopentasiloxane manufactured by Shin-Etsu Chemical Co., Ltd. |
| (Note 5) | Polymethylsilsesquioxane manufactured by Shin-Etsu Chemical Co., Ltd. |
| (Note 6) | An (acrylates/ethylhexyl acrylate/dimethicone methacrylate) copolymer manufactured by Shin-Etsu Chemical Co., Ltd. |
| (Note 7) | KF-9909-treated color inorganic pigments W: white, R: red, Y: yellow, B: black each manufactured by Shin-Etsu Chemical Co., Ltd. |

### (Production Method)

A: The components 10 to 12 were dispersed with a rolling mill.
B: The components 1 to 9 were uniformly mixed.
C: The components 13 to 17 were uniformly mixed.
D: The mixture prepared in C was added to the mixture prepared in B and was emulsified, and the mixture prepared in A was added thereto to prepare a W/O cream foundation.

The W/O cream foundation was a cosmetic with high usability, high applicability, high emulsion stability, and a good soft focus effect.

### [Example 23] O/W Foundation Cream

| Composition | | % |
|---|---|---|
| 1. | Production Example 2 (component a: 15%, component b: 85%) | 0.5 |
| 2. | KMP-592 (Note 1) | 10 |
| 3. | Polysorbate-60 | 2 |
| 4. | A sodium acrylate-sodium acryloyldimethyltaurate copolymer composition (Note 2) | 1 |
| 5. | 5% aqueous solution of (acryloyldimethyltaurine ammonium/VP) copolymer (Note 3) | 15 |
| 6. | Butylene glycol | 10 |
| 7. | Xylitol | 3 |
| 8. | Pentylene glycol | 1 |
| 9. | Caprylyl glycol | 0.2 |
| 10. | Purified water | 57.3 |
| Total | | 100.0 |

| | |
|---|---|
| (Note 1) | (Methyl/phenyl) polysilsesquioxane manufactured by Shin-Etsu Chemical Co., Ltd. |
| (Note 2) | SIMULGEL EG manufactured by SEPPIC |
| (Note 3) | 5% aqueous solution of Aristoflex AVC manufactured by Clariant |

### (Production Method)

A: The components 3 to 10 were uniformly mixed.
B: The components 1 and 2 were uniformly mixed.
C: The mixture prepared in B was added to the mixture prepared in A and was emulsified to prepare an O/W foundation cream.

The O/W foundation cream was a cosmetic with high usability, applicability, and stability.

### [Example 24] O/W Cream

| Composition | | % |
|---|---|---|
| 1. | Production Example 1 (component a: 30%, component b: 70%) | 5 |
| 2. | KSG-15 (Note 1) (component b: 90% to 96%) | 35 |
| 3. | Isotridecyl isononanoate | 10 |
| 4. | BG | 7 |
| 5. | KF-6100 (Note 2) | 0.7 |
| 6. | PEG-60 hydrogenated castor oil | 0.5 |
| 7. | A sodium acrylate-sodium acryloyldimethyltaurate copolymer composition (Note 3) | 0.8 |
| 8. | 5% aqueous solution of (Acryloyldimethyltaurine ammonium/VP) copolymer (Note 4) | 10 |
| 9. | PCA-Na aqueous solution | 0.5 |
| 10. | Ethylhexyl glycerol | 0.1 |
| 11. | Purified water | Balance |
| Total | | 100.0 |

| | |
|---|---|
| (Note 1) | A mixture of cyclopentasiloxane 90% to 96% + (dimethicone/vinyl dimethicone) crosspolymer 4% to 10% manufactured by Shin-Etsu Chemical Co., Ltd. |
| (Note 2) | Polyglyceryl-3 disiloxane dimethicone manufactured by Shin-Etsu Chemical Co., Ltd. |
| (Note 3) | SIMULGEL EG manufactured by SEPPIC |
| (Note 4) | 5% aqueous solution of Aristoflex AVC manufactured by Clariant |

### (Production Method)

A: The components 4 to 11 were uniformly mixed.
B: The components 1 to 3 were uniformly mixed.
C: The mixture prepared in B was added to the mixture prepared in A and was uniformly mixed to prepared O/W cream.

The O/W cream was a cosmetic with high usability, applicability, and stability.

### [Example 25] W/O Concealer

| Composition | | % |
|---|---|---|
| 1. | Production Example 1 (component a: 30%, component b: 70%) | 6 |
| 2. | KSG-43 (Note 1) | 25 |
| 3. | KSG-15 (Note 2) (component b: 90% to 96%) | 15 |
| 4. | KSG-19 (Note 3) | 5 |
| 5. | KF-6104 (Note 4) | 1 |
| 6. | TMF-1.5 (Note 5) (component b) | 13 |
| 7. | Hexyl laurate | 5 |
| 8. | Mg sulfate | 0.4 |
| 9. | Na citrate | 0.1 |
| 10. | Glycerol | 2 |
| 11. | Pentylene glycol | 2 |
| 12. | Water | 25.5 |
| Total | | 100.0 |

| | |
|---|---|
| (Note 1) | A mixture of triethylhexanoin 65% to 75% + (vinyl dimethicone/lauryl dimethicone) crosspolymer 25% to 35% manufactured by Shin-Etsu Chemical Co., Ltd. |
| (Note 2) | A mixture of cyclopentasiloxane 90% to 96% + (dimethicone/vinyl dimethicone) crosspolymer 4% to 10% manufactured by Shin-Etsu Chemical Co., Ltd. |
| (Note 3) | A mixture of dimethicone (6 cs) 80% to 90% + (dimethicone/vinyl dimethicone) crosspolymer 10% to 20% manufactured by Shin-Etsu Chemical Co., Ltd. |
| (Note 4) | Polyglyceryl-3 polydimethylsiloxyethyl dimethicone manufactured by Shin-Etsu Chemical Co., Ltd. |
| (Note 5) | Methyl trimethicone manufactured by Shin-Etsu Chemical Co., Ltd. |

### (Production Method)

A: The components 1 to 7 were uniformly mixed.
B: The components 8 to 12 were uniformly mixed.
C: While stirring, the mixture prepared in B was gradually added to the mixture prepared in A and was emulsified to prepare a W/O concealer.

The W/O concealer was a cosmetic with an emollient feel, high usability, and a good soft focus effect.

### [Example 26] W/O Foundation

| Composition | | % |
|---|---|---|
| 1. | Production Example 2 (component a: 15%, component b: 85%) | 13 |
| 2. | KSG-210 (Note 1) | 5 |
| 3. | KSG-270 (Note 2) | 5 |
| 4. | KF-4422 (Note 3) (component b) | 11 |
| 5. | KF-56A (Note 4) | 9 |
| 6. | KTP-09W, R, Y, B (Note 5) | 10 |
| 7. | KMP-591 (Note 6) | 1 |
| 8. | BG | 1 |
| 9. | Glycerol | 6 |
| 10. | Sodium citrate | 0.2 |
| 11. | Sodium chloride | 0.5 |
| 12. | Water | 38.3 |
| Total | | 100.0 |

| | |
|---|---|
| (Note 1) | A mixture of dimethicone 70% to 80% + (dimethicone/(PEG-10/15)) crosspolymer 20% to 30% manufactured by Shin-Etsu Chemical Co., Ltd. |
| (Note 2) | A mixture of diphenylsiloxyphenyl trimethicone 75% to 85% + (dimethicone/(PEG-10/15)) crosspolymer 15% to 25% manufactured by Shin-Etsu Chemical Co., Ltd. |
| (Note 3) | Ethyl methicone manufactured by Shin-Etsu Chemical Co., Ltd. |
| (Note 4) | Diphenylsiloxyphenyl trimethicone manufactured by Shin-Etsu Chemical Co., Ltd. |
| (Note 5) | KF-9909-treated color inorganic pigments W: white, R: red, Y: yellow, B: black each manufactured by Shin-Etsu Chemical Co., Ltd. |
| (Note 6) | Polymethylsilsesquioxane manufactured by Shin-Etsu Chemical Co., Ltd. |

### (Production Method)

A: The components 1 to 7 were uniformly mixed.
B: The components 8 to 12 were uniformly mixed.
C: The mixture prepared in B was added to the mixture prepared in A and was emulsified to prepare a W/O foundation.

The W/O foundation was a cosmetic with an emollient feel, high usability, and a good soft focus effect.

### [Example 27] W/O Sunscreen Cosmetic

| Composition | | % |
|---|---|---|
| 1. | Dimethicone 1.5 cs(component b) | 20 |
| 2. | Disteardimonium hectorite | 0.6 |
| 3. | Glyceryl (behenate/eicosanedicarboxylate) | 0.6 |
| 4. | Dimethicone 6 cs | 2 |
| 5. | Isostearyl neopentanoate | 6 |
| 6. | Silylated silica | 0.6 |
| 7. | Production Example 1 (component a: 30%, component b: 70%) | 0.6 |
| 8. | SPD-T5L (Note 1) (component b: 50%) | 6 |
| 9. | Ethylhexyl methoxycinnamate | 7.5 |
| 10. | KF-6028 | 2 |
| 11. | SPD-Z7L (Note 2) (component b: 35%) | 15 |
| 12. | KSP-105 | 2 |
| 13. | BG | 5 |
| 14. | Glycerol | 2 |
| 15. | Sodium chloride | 0.5 |
| 16. | Sodium citrate | 0.2 |
| 17. | Water | 29.4 |
| Total | | 100.0 |

| | |
|---|---|
| (Note 1) | A metallic soap-treated fine titanium dioxide particle dispersion (45%) manufactured by Shin-Etsu Chemical Co., Ltd. |
| (Note 2) | An alkylsilane-treated fine zinc oxide particle dispersion (60%) manufactured by Shin-Etsu Chemical Co., Ltd. |

### (Production Method)

A: The components 1 to 12 were uniformly mixed.
B: The components 13 to 17 were uniformly mixed.
C: The mixture prepared in B was added to the mixture prepared in A and was emulsified to prepare a W/O sunscreen cosmetic.

The resulting W/O sunscreen cosmetic was a cosmetic with an emollient feel, high usability, and a good soft focus effect.

### [Example 28] W/O Hair Cosmetic

| Composition | | % |
|---|---|---|
| 1. | Production Example 1 (component a: 30%, component b: 70%) | 3 |
| 2. | KSG-16 | 4 |
| 3. | KF-6017 | 0.1 |
| 4. | Dimethiconol | 15 |
| 5. | (Caprylic/capric) triglyceride | 3 |
| 6. | BG | 10 |
| 7. | Ethanol | 5 |
| 8. | Sodium citrate | 0.2 |
| 9. | Sodium chloride | 0.5 |
| 10. | Water | 59.2 |
| Total | | 100.0 |

### (Production Method)

A: The components 1 to 5 were uniformly mixed.
B: The components 7 to 10 were uniformly mixed.
C: The mixture prepared in B was added to the mixture prepared in A and was emulsified to prepare a W/O hair cosmetic.

The W/O hair cosmetic was a cosmetic with an emollient feel, high usability, and a good soft focus effect.

### [Example 29] W/O Liquid Foundation

| Composition | | % |
|---|---|---|
| 1. | Production Example 1 (component a: 30%, component b: 70%) | 3.5 |
| 2. | KSG-43 (Note 1) | 5 |
| 3. | KF-6104 (Note 2) | 2.5 |
| 4. | Organic modified clay mineral | 0.8 |
| 5. | Dimethyl polysiloxane (2 cs) (component b) | 21 |
| 6. | Ethylhexyl palmitate | 7 |
| 7. | (Caprylic/capric) triglyceride | 5 |
| 8. | KSP-101 (Note 3) | 2 |
| 9. | KF-56A (Note 4) | 7 |
| 10. | KF-6106 (Note 5) | 1 |
| 11. | KTP-09W (Note 6) | 10 |
| 12. | KTP-09R (Note 6) | 0.2 |
| 13. | KTP-09Y (Note 6) | 0.5 |
| 14. | KTP-09B (Note 6) | 0.1 |
| 15. | Metallic soap-treated fine titanium dioxide particles | 10 |
| 16. | BG | 5 |
| 17. | Na citrate | 0.2 |
| 18. | Na chloride | 1 |
| 19. | Water | 18.2 |
| Total | | 100.0 |

| | |
|---|---|
| (Note 1) | A mixture of triethylhexanoin 65% to 75% + (vinyl dimethicone/lauryl dimethicone) crosspolymer 25% to 35% manufactured by Shin-Etsu Chemical Co., Ltd. |
| (Note 2) | Polyglyceryl-3 polydimethylsiloxyethyl dimethicone manufactured by Shin-Etsu Chemical Co., Ltd. |
| (Note 3) | A (vinyl dimethicone/methicone silsesquioxane) crosspolymer manufactured by Shin-Etsu Chemical Co., Ltd. |
| (Note 4) | Diphenylsiloxyphenyl trimethicone manufactured by Shin-Etsu Chemical Co., Ltd. |
| (Note 5) | Lauryl polyglyceryl-3 polydimethylsiloxyethyl dimethicone manufactured by Shin-Etsu Chemical Co., Ltd. |
| (Note 6) | KF-9909-treated color inorganic pigments W: white, R: red, Y: yellow, B: black each manufactured by Shin-Etsu Chemical Co., Ltd. |

### (Production Method)

A: The components 9 to 15 were dispersed with a three-roll mill.
B: The components 1 to 8 were uniformly mixed.
C: The components 16 to 19 were uniformly mixed.
D: The mixture prepared in C was added to the mixture prepared in B and was emulsified, and the mixture prepared in A was added thereto to prepared a W/O liquid foundation.

The W/O liquid foundation had high usability, applicability, and storage stability.

### [Example 30] W/O Solid Foundation

| Composition | | % |
|---|---|---|
| 1. | Production Example 1 (component a: 30%, component b: 70%) | 5 |
| 2. | KSG-19 (Note 1) | 3 |
| 3. | KF-6105 (Note 2) | 2 |
| 4. | SPD-T7 (Note 3) (component b: 49%) | 10 |
| 5. | KF-56A (Note 4) | 7.35 |
| 6. | Ceresin | 5 |
| 7. | Isotridecyl isononanoate | 2 |
| 8. | AES-3083 (Note 5) treated pigment | 7.9 |
| 9. | KP-545 (Note 6) | 0.3 |
| 10. | BG | 7 |
| 11. | Glycerol | 5 |
| 12. | Na citrate | 0.2 |
| 13. | Na chloride | 1 |
| 14. | Water | Balance |
| Total | | 100.0 |

| | |
|---|---|
| (Note 1) | A mixture of dimethicone (6 cs) 70% to 80% + (dimethicone/polyglycerin-3) crosspolymer 20% to 30% manufactured by Shin-Etsu Chemical Co., Ltd. |
| (Note 2) | Lauryl polyglyceryl-3 polydimethylsiloxyethyl dimethicone manufactured by Shin-Etsu Chemical Co., Ltd. |
| (Note 3) | A cyclopentasiloxane dispersion of 45% fine titanium dioxide particles manufactured by Shin-Etsu Chemical Co., Ltd. |
| (Note 4) | Diphenylsiloxyphenyl trimethicone manufactured by Shin-Etsu Chemical Co., Ltd. |
| (Note 5) | Triethoxycaprylylsilane manufactured by Shin-Etsu Chemical Co., Ltd. |
| (Note 6) | An (acrylates/dimethicone) copolymer manufactured by Shin-Etsu Chemical Co., Ltd. |

### (Production Method)

A: The components 7 to 9 were dispersed with a three-roll mill.
B: The components 1 to 6 were dissolved at 90°C, and A was then added thereto and was uniformly mixed.
C: The components 10 to 14 were uniformly mixed.
D: The mixture prepared in C was added to the mixture prepared in B and was emulsified to prepare a W/O solid foundation.

The W/O solid foundation had high usability, applicability, and storage stability.

### [Example 31] Mousse Foundation

| Composition | | % |
|---|---|---|
| 1. | KSG-16 (Note 1) | 20 |
| 2. | Production Example 2 (component a: 15%, component b: 85%) | 12 |
| 3. | KF-7312T (Note 2) | 8 |
| 4. | Neopentyl glycol diethylhexanoate | 4 |
| 5. | Dimethicone (2 cs) | Balance |
| 6. | Macadamia nut oil | 0.5 |
| 7. | KSP-101 (Note 3) | 2 |
| 8. | Poly(methyl methacrylate) | 3 |
| 9. | Dimethicone (6 cs) | 10 |
| 10. | Silicone-treated titanium oxide (Note 4) | 5 |
| 11. | Metallic soap-treated fine titanium dioxide particles | 10 |
| 12. | Silicone-treated red iron oxide (Note 4) | 0.2 |
| 13. | Silicone-treated yellow iron oxide (Note 4) | 0.5 |
| 14. | Silicone-treated black iron oxide (Note 4) | 0.1 |
| 15. | Silicone-treated talc (Note 4) | 3 |
| 16. | Silicone-treated black mica (Note 4) | 5 |
| Total | | 100.0 |

| | |
|---|---|
| (Note 1) | A mixture of (dimethicone/vinyl dimethicone) crosspolymer (label name) 25% and dimethicone (label name) 75% manufactured by Shin-Etsu Chemical Co., Ltd. |
| (Note 2) | Trimethylsiloxysilicic acid (label name (INCI: Trimethylsiloxysilicate) manufactured by Shin-Etsu Chemical Co., Ltd. |
| (Note 3) | A (vinyl dimethicone/methicone silsesquioxane) crosspolymer manufactured by Shin-Etsu Chemical Co., Ltd. (label name) |
| (Note 4) | manufactured by Shin-Etsu Chemical Co., Ltd., treated with KF-9908 |

### (Production Method)

A: The components 9 to 16 were dispersed with a three-roll mill.
B: The components 1 to 8 were uniformly mixed.
C: The mixture prepared in A was added to the mixture prepared in B and was uniformly mixed to prepare a mousse foundation.

The mousse foundation had high usability, applicability, and storage stability.

### [Example 32] Powder Foundation

| Composition | | % |
|---|---|---|
| 1. | Ethylhexyl methoxycinnamate | 4 |
| 2. | KF-56A (Note 1) | 4.5 |
| 3. | Triethylhexanoin | 1.5 |
| 4. | KP-561P (Note 2) | 1 |
| 5. | Production Example 3 (component a: 60%, component b: 40%) | 1 |
| 6. | Silicone-treated mica (Note 3) | 30 |
| 7. | Ba sulfate | 10 |
| 8. | KSP-300 (Note 4) | 3 |
| 9. | KMP-590 (Note 5) | 5 |
| 10. | Silicone-treated talc (Note 3) | Balance |
| 11. | Silicone-treated titanium dioxide (Note 3) | 8 |
| 12. | Silicone-treated yellow iron oxide (Note 3) | 1.0 |
| 13. | Silicone-treated red iron oxide (Note 3) | 0.4 |
| 14. | Silicone-treated black iron oxide (Note 3) | 0.2 |
| Total | | 100.0 |

| | |
|---|---|
| (Note 1) | Diphenylsiloxyphenyl trimethicone (label name) manufactured by Shin-Etsu Chemical Co., Ltd. |
| (Note 2) | An (acrylates/stearyl acrylate/dimethicone methacrylate) copolymer (label name) manufactured by Shin-Etsu Chemical Co., Ltd. |
| (Note 3) | manufactured by Shin-Etsu Chemical Co., Ltd., treated with KF-9909 |
| (Note 4) | A (diphenyl dimethicone/vinyl diphenyl dimethicone/silsesquioxane) crosspolymer (label name) manufactured by Shin-Etsu Chemical Co., Ltd. |
| (Note 5) | Polymethyl silsesquioxane (label name) manufactured by Shin-Etsu Chemical Co., Ltd. |

### (Production Method)

A: The components 1 to 5 were heated to 50°C, were uniformly mixed, and were cooled to room temperature.
B: The components 6 to 14 were uniformly mixed.
C: The mixture prepared in A was added to B and was uniformly mixed in a Henschel mixer. The resulting powder was passed through a mesh and was then molded into a metal plate using a mold to prepare a powder foundation.

The powder foundation had high usability, applicability, and storage stability.

## Claims

1. A cosmetic comprising a silicone gel in which the following component (a) is swollen with the following component (b):
(a) a cross-linked glycerol-modified silicone in which dimethyl polysiloxane is cross-linked with a glycerol chain, and
(b) an oil agent with a kinematic viscosity in the range of 0.5 to 5 mm²/s at 25°C.

2. The cosmetic according to claim 1, wherein the component (b) is a silicone oil with a kinematic viscosity in the range of 0.5 to 5 mm²/s at 25°C.

3. The cosmetic according to claim 2, wherein the component (b) is caprylyl methicone.

4. The cosmetic according to claim 1, wherein the component (a) is a (dimethicone/polyglycerin-3) crosspolymer.

5. The cosmetic according to claim 1, further comprising (c) an ultraviolet absorber: 3.5% to 20% by mass.

6. The cosmetic according to claim 1, further comprising (d) a powder: 1% to 40% by mass.

7. The cosmetic according to any one of claims 1 to 6, wherein the cosmetic is a makeup cosmetic.
